# EUROPEAN PATENT APPLICATION

(11) **EP 4 692 089 A1**
(43) Date of publication of application: **11.02.2026**
(21) Application number: 24784030.9
(22) Date of filing: 13.03.2024
(51) Int. Cl.: C07D 487/04, C07D 401/04, A61P 25/00, A61K 31/444

(54) **NITROGEN-CONTAINING HETEROCYCLIC COMPOUND AS AAK1 INHIBITOR**

(30) Priority: 07.04.2023 CN 202310408679; 06.03.2024 CN 202410255161
(71) Applicant: Alicorn Pharmaceutical Co., Ltd., Haining Jiaxing, Zhejiang 314400 (CN)
(72) Inventor: LU, Pingbo, Jiaxing, Zhejiang 314400 (CN); YANG, Jiale, Jiaxing, Zhejiang 314400 (CN); LU, Xin, Jiaxing, Zhejiang 314400 (CN); YANG, Wei, Jiaxing, Zhejiang 314400 (CN); WANG, Zhi, Jiaxing, Zhejiang 314400 (CN); YAO, Yueyue, Jiaxing, Zhejiang 314400 (CN); ZHAO, Fang, Jiaxing, Zhejiang 314400 (CN)
(74) Representative: Vitillo, Giuseppe
(86) International application number: PCT/CN2024/081339
(87) International publication number: WO 2024/207945

(57) **Abstract**

The present invention relates to use of a nitrogen-containing heterocyclic compound or a pharmaceutically acceptable salt thereof for inhibiting activity of adaptor associated kinase 1 (AAK1 kinase) in a medicament for treating or preventing a disease or a disorder mediated by the activity of AAK1. Specifically, the present invention provides a compound of formula I, a stereoisomer thereof, or a pharmaceutically acceptable salt thereof, where a definition of each group is as described in the specification. The compound has relatively high AAK1 inhibitory activity. (FIG. 1)

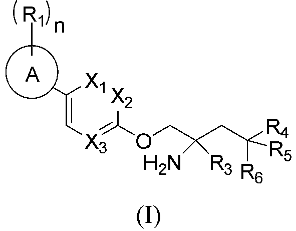

## Description

### FIELD OF TECHNOLOGY

The present invention belongs to the field of pharmaceuticals, and specifically relates to a nitrogen-containing heterocyclic compound capable of inhibiting activity of adaptor associated kinase 1 (AAK1), a composition containing the above compound, and use of both in preparation of a medicament for treating a disease or a disorder mediated by the activity of adaptor associated kinase 1.

### BACKGROUND

Adaptor associated kinase 1 (AAK1) is a member of the Ark1/Prk1 family of serine/threonine kinases. AAK1 mRNA exists in two splice forms, referred to as the short form and the long form. The long form is dominant and is highly expressed in the brain and heart (Henderson and Conner, Mol. Biol. Cell. 2007, 18, 2698-2706). AAK1 is enriched in synaptosomal preparations and colocalizes with endocytic structures in cultured cells. AAK1 regulates clathrin-coated endocytosis, which is an important process in synaptic vesicle recycling and receptor-mediated endocytosis. AAK1 binds to the AP2 complex, a heterotetramer that links receptor cargo to clathrin coats. Binding of clathrin to AAK1 stimulates the activity of AAK1 kinase (Conner et al., Traffic 2003, 4, 885-890; Jackson et al., J. Cell. Biol. 2003, 163, 231-236). AAK1 phosphorylates the mu-2 subunit of AP-2, which promotes the binding of mu-2 to tyrosine-containing sorting motifs on cargo receptors (Ricotta et al., J. Cell Bio. 2002, 156, 791-795; Conner and Schmid, J. Cell Bio. 2002, 156, 921-929). Mu2 phosphorylation is not essential for receptor uptake, but phosphorylation increases the efficiency of internalization (Motely et al., Mol. Biol. Cell. 2006, 17, 5298-5308).

AAK1 has been identified as an inhibitor of neuregulin-1/ErbB4 signaling in PC12 cells. Loss of AAK1 expression via RNA interference-mediated gene silencing or treatment with the kinase inhibitor K252a (which inhibits the activity of AAK1 kinase) leads to enhanced neuregulin-1-induced neurite outgrowth. These treatments result in increased ErbB4 expression and increased accumulation of ErbB4 in or near the plasma membrane (Kuai et al., Chemistry and Biology 2011, 18, 891-906). NRG1 and ErbB4 are putative schizophrenia susceptibility genes (Buonanno, Brain Res. Bull. 2010, 83, 122-131). Single nucleotide polymorphisms (SNPs) in both genes are associated with various schizophrenia endophenotypes (Greenwood et al., Am. J. Psychiatry 2011, 168, 930-946). Neuregulin 1 and ErbB4 knockout (KO) mouse models have shown schizophrenia-related morphological changes and behavioral phenotypes (Jaaro-Peled et al., Schizophrenia Bulletin 2010, 36, 301-313; Wen et al., Proc. Natl. Acad. Sci. USA. 2010, 107, 1211-1216). Furthermore, a single nucleotide polymorphism in an intron of the AAK1 gene is associated with the age of onset of Parkinson's disease (Latourelle et al., BMC Med. Genet. 2009, 10, 98). These results indicate that inhibiting the activity of AAK1 can be used for the treatment of schizophrenia, cognitive deficits in schizophrenia, Parkinson's disease, neuropathic pain, bipolar disorder, and Alzheimer's disease.

Documents such as CN106458994A, CN108290843A, and WO2023284838A have disclosed many AAK1 inhibitor compounds. J. Med. Chem. 2022, 65, 4457-4480 has disclosed the compound BMS-986176/LX-9211 for inhibiting the activity of AAK1.

### SUMMARY

### Summary of the Invention

The inventors have conducted extensive research and found that the compound and the stereoisomer thereof or the pharmaceutically acceptable salt thereof provided by the present invention possess a strong AAK1 inhibitory function, overcoming the deficiencies in AAK1 inhibitory function existing in the prior art. The nitrogen-containing heterocyclic compound provided by the present invention exhibits a good inhibitory effect on AAK1.

The present invention solves the above technical problems through the following technical solutions.

In one aspect, the present invention provides a compound of formula I, a stereoisomer thereof, or a pharmaceutically acceptable salt thereof: 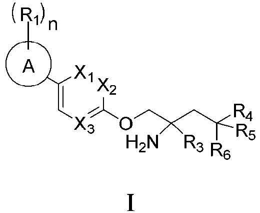 where
X₁, X₂, and X₃ are each independently selected from N or CR₂;
a ring A is selected from: 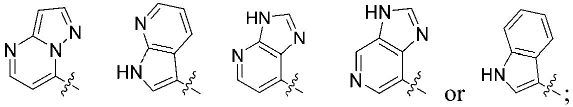
R₁, R₂, and R₃ are each independently selected from hydrogen, amino, -CO₂H, halogen, fluoromethyl, difluoromethyl, trifluoromethyl, cyano, formamido, C₁₋₆ alkyl, deuterated C₁₋₆ alkyl, halogenated C₁₋₆ alkyl, C₁₋₆ alkoxy, deuterated C₁₋₆ alkoxy, halogenated C₁₋₆ alkoxy, hydroxy C₁₋₆ alkyl, C₃₋₆ cycloalkyl, or 4-6 membered heterocycloalkyl containing 1-3 heteroatoms selected from N, S, or O;
R₄, R₅, and R₆ are each independently selected from hydrogen, deuterium, halogen, amino, cyano, hydroxy, alkenyl, fluoromethyl, difluoromethyl, trifluoromethyl, C₁₋₆ alkyl, halogenated C₁₋₆ alkyl, C₁₋₆ alkoxy, halogenated C₁₋₆ alkoxy, deuterated C₁₋₆ alkyl, deuterated C₁₋₆ alkoxy, C₃₋₆ cycloalkyl, hydroxy C₁₋₆ alkyl, or 4-6 membered heterocycloalkyl containing 1-3 heteroatoms selected from N, S, or O;
or R₄ and R₅, together with carbon atoms to which they are each attached, form C₃₋₆ cycloalkyl, 4-6 membered heterocycloalkyl containing 1-3 heteroatoms selected from N, S, or O, or a double bond; and
n is selected from 0, 1, 2, 3, or 4, and when the n is greater than 1, R₁ is the same or different.

As a preferred technical solution, R₁ is selected from hydrogen, fluorine, chlorine, -CO₂H, cyano, methoxy, methyl, difluoromethyl, trifluoromethyl, or formamido.

As a preferred technical solution, R₂ is selected from hydrogen, methyl, cyano, difluoromethyl, trifluoromethyl, and cyclopropyl.

As a preferred technical solution, R₃ is selected from methyl, and R₄, R₅, and R₆ are each independently selected from hydrogen, methyl, halogen, alkenyl, or 4-6 membered heterocycloalkyl containing 1-3 heteroatoms selected from N, S, or O.

As a preferred technical solution, R₄ and R₅, together with the carbon atoms to which they are each attached, form 4-6 membered heterocycloalkyl containing 1-3 heteroatoms selected from N, S, or O, or a double bond.

As a preferred technical solution, 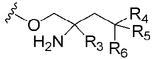 is selected from the following groups:

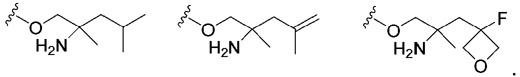

In a second aspect, the present invention provides a compound of formula I-1, a stereoisomer thereof, or a pharmaceutically acceptable salt thereof: 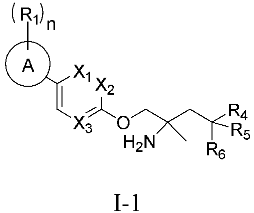 where R₁, R₂, a ring A, n, R₄, R₅, R₆, X₁, X₂, and X₃ are as defined above.

In a third aspect, the present invention provides a compound of formula I-2, a stereoisomer thereof, or a pharmaceutically acceptable salt thereof: 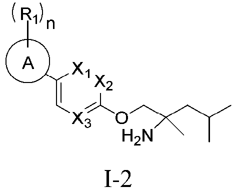 where X₁, X₂, X₃, R₁, R₂, a ring A, and n are as defined above.

In the fourth aspect, the present invention provides a compound of formula I-3, I-4, I-5, I-6, I-7, or I-8, a stereoisomer thereof, or a pharmaceutically acceptable salt thereof: 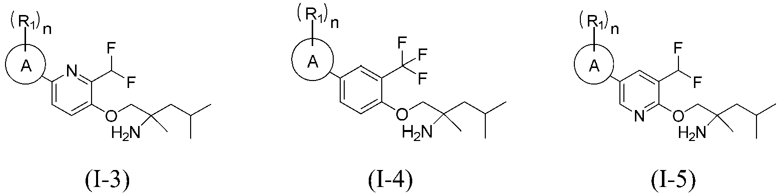 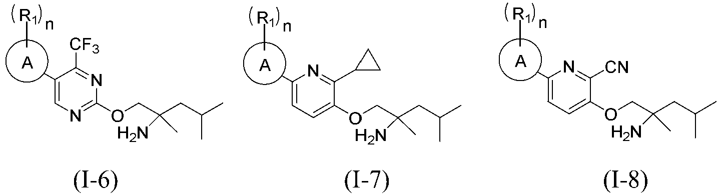 where R₁, a ring A, and n are as defined above.

As a preferred technical solution, the compound provided by the present invention includes, but is not limited to, the following compounds:

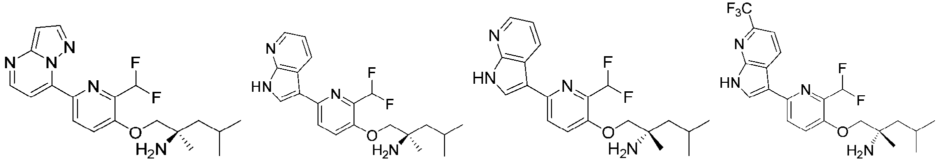

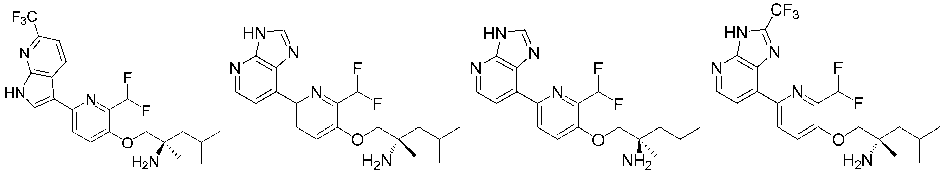

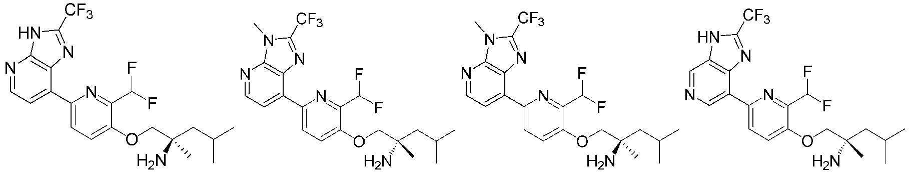

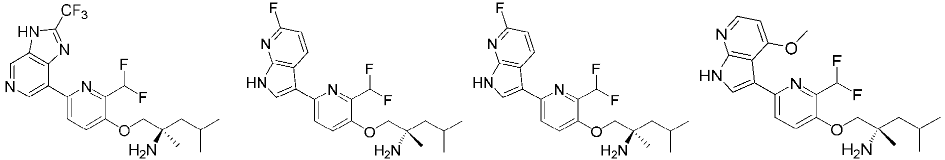

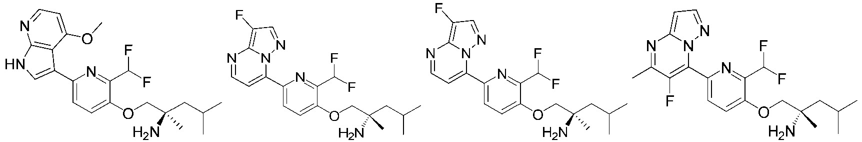

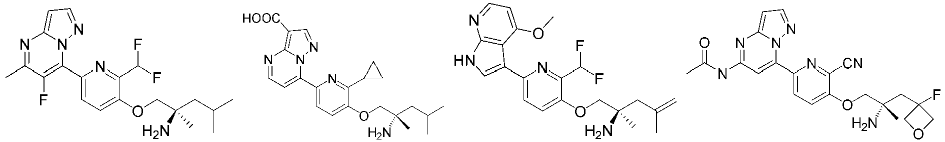

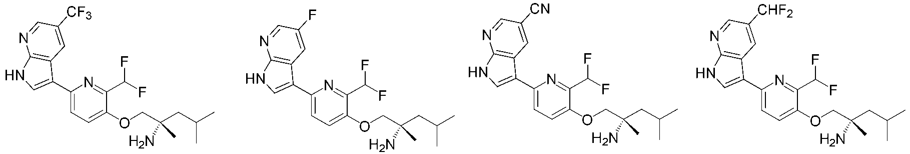

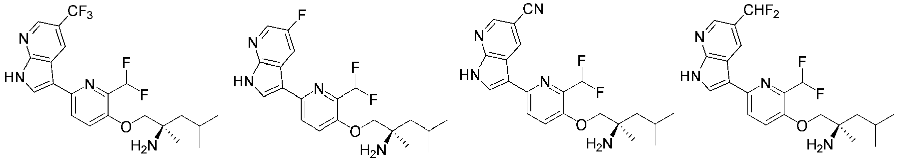

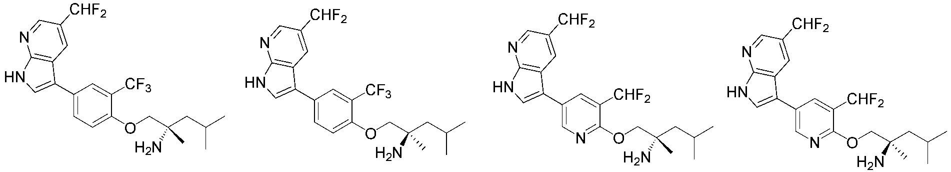

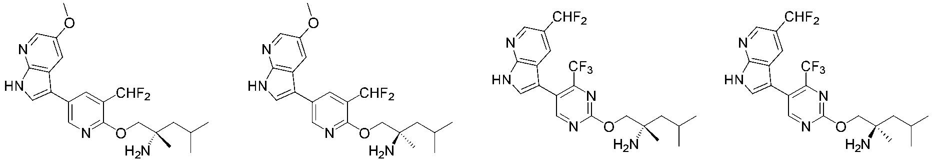

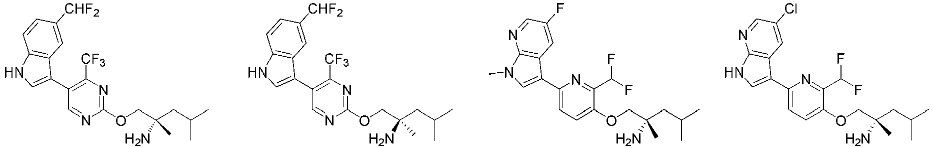

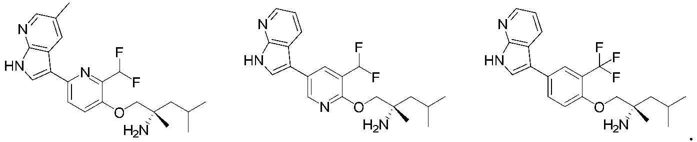

In a fifth aspect, the present invention provides a pharmaceutical composition, containing the compound, the stereoisomer thereof, or the pharmaceutically acceptable salt thereof according to any one of the foregoing technical solutions, and a pharmaceutically acceptable carrier.

The present invention further provides use of the compound, the stereoisomer thereof, or the pharmaceutically acceptable salt thereof, or a pharmaceutical composition according to any one of the foregoing technical solutions in preparation of a medicament for treating a disease or a disorder mediated by activity of adaptor associated kinase 1.

As a preferred technical solution, the disease or disorder is selected from Alzheimer's disease, bipolar disorder, Parkinson's disease, schizophrenia, diabetic peripheral neuropathic pain, or postherpetic neuralgia.

A dosage of the compound, the stereoisomer thereof, or the pharmaceutically acceptable salt thereof in the present invention is calculated in a form of free base in each case.

The inventors have found that such compounds are efficient AAK1 inhibitors with strong AAK1 inhibitory activity, and can be used to prepare a medicament for treating a disease or a disorder mediated by activity of AAK1, including Alzheimer's disease, bipolar disorder, Parkinson's disease, schizophrenia, diabetic peripheral neuropathic pain, postherpetic neuralgia, fibromyalgia, or peripheral neuropathy, etc. The present invention is completed based on the above findings.

### Detailed Description of the Invention

The various aspects and features of the present invention are further described below.

All documents cited in the present invention are incorporated herein by reference in their entirety, and if the meanings expressed by these documents are inconsistent with the present invention, the expressions of the present invention shall prevail. In addition, the various terms and phrases used in the present invention have the general meanings well known to those skilled in the art; even so, the present invention still intends to provide more detailed descriptions and explanations of these terms and phrases herein. If the mentioned terms and phrases are inconsistent with their well-known meanings, the meanings expressed in the present invention shall prevail. The following are definitions of various terms used in the present invention, which apply to the terms used throughout the specification of the present application, unless otherwise specified in specific cases.

The compound according to the present invention may exist in a tautomeric form, and thus the present invention includes all tautomeric forms.

The compound of the present invention has an asymmetric center. Compounds containing asymmetrically substituted atoms in the present invention can be separated into optically active or racemic forms. Those skilled in the art know how to prepare optically the active form, such as through resolution of racemates or synthesis from optically active starting materials. Unless specific stereochemistry or isomeric forms are specifically indicated, the present invention includes all chiral forms, diastereoisomers, and racemates. A method for preparing the compound of the present invention and an intermediate thereof are part of the present invention. All tautomers of the compound of the present invention are also part of the present invention.

The singular forms "a", "an" and "the" include plural referents unless the context clearly indicates otherwise.

As used herein, the term "optional" or "optionally" means that the event or circumstance described hereinafter may or may not occur, and the description includes instances where the event or circumstance occurs and instances where it does not. For example, "optionally substituted alkyl" refers to the event or circumstance where the alkyl group may be substituted and where the alkyl group is unsubstituted.

The term "substituted" means a moiety having substituents that replace hydrogen on one or more carbons of the main chain. It should be understood that "substituted" or "substituted by" includes the implicit condition that in such substitution, the permissible valences of the substituted atom and the substituent are consistent, and that the substitution results in a stable compound, which did not, for example, spontaneously undergo transformation, for example, by rearrangement, cyclization, elimination, and the like. As used herein, the term "substituted" is intended to include all permissible substituents of organic compounds. In a broad sense, permissible substituents include acyclic and cyclic, branched and unbranched, carbocyclic and heterocyclic, aromatic and non-aromatic substituents of organic compounds. For appropriate organic compounds, the permissible substituents may be one or more, and they may be the same or different. For the objectives of the present invention, heteroatoms such as nitrogen may have hydrogen substituents and/or any permissible substituents of the organic compounds described herein that satisfy the valencies of the heteroatoms. Substituents may include any of the substituents described herein, such as halogens, hydroxy, alkyl, alkoxy, amino, cyano, heteroaryl, heterocyclic, and the like. Those skilled in the art will understand that substituents themselves may be substituted, where appropriate.

As described herein, the terms "halogen," "halo," and the like refer to fluorine, chlorine, bromine, or iodine, particularly to fluorine, chlorine, or bromine, and particularly preferably to fluorine or chlorine.

The expression "C_{x-y} group" refers to a group containing x to y carbon atoms. For example, "C₁₋₆" refers to an alkyl group containing 1 to 6 carbon atoms.

As described herein, the term "alkyl" refers to a straight or branched alkyl group having the specified number of carbon atoms, and may include subgroups thereof. For example, reference to "C₁-C₆ alkyl" may also include groups of subranges represented by C₁-C₄ alkyl, C₁-C₃ alkyl, C₂-C₆ alkyl, C₂-C₄ alkyl, and the like, as well as specific groups such as methyl, ethyl, n-propyl, isopropyl, n-butyl, sec-butyl, tert-butyl, pentyl, hexyl, and the like.

"Halogenated C₁₋₆ alkyl" refers to an alkyl group containing 1 to 6 carbon atoms, in which one or more hydrogen atoms are substituted by one or more halogen atoms (such as fluorine, chlorine, bromine, and iodine). The upper limit of the number of halogen substituents is equal to the total number of hydrogen atoms that can be substituted in the alkyl group. Without particular limitation, the number of halogen substituents is any integer between 1 and the upper limit, preferably 1 to 5 halogen substituents, 1 to 3 halogen substituents, 1 to 2 halogen substituents, or 1 halogen substituent. When the number of halogen substituents is more than 1, they may be substituted by the same or different halogens, including but not limited to -CF₃, -CH₂Cl, -CH₂CF₃, -CCl₂, CF₃, and the like.

"Hydroxyalkyl" refers to an alkyl group substituted by a hydroxy group, where the alkyl group is as defined above.

The term "alkoxy" is a conventional expression referring to an alkyl group attached to the remainder of the molecule through an oxygen atom.

It denotes an alkyl group containing an oxygen atom. Examples of such groups include methoxy, ethoxy, propoxy, and the like.

The term "halogenated alkyl" refers to an alkyl group, as defined above, linked to the remainder of the molecule via a halogenated linkage. For example, C1-6 halogenated alkyl refers to an alkyl group having 1 to 6 carbon atoms, or 1 to 3 carbon atoms, linked to the remainder of the molecule via a halogenated linkage. Preferred halogenated alkyl groups include, but are not limited to, -CH₂Cl, -CHCl₂, -CF₃, and the like.

"Halogenated alkoxy" refers to -O-halogenated alkyl. Without particular limitation, it refers to -O-halogenated C₁₋₈ alkyl, preferably -O-halogenated C₁₋₆ alkyl, more preferably -O-halogenated C₁₋₄ alkyl, and further preferably -O-halogenated C₁₋₂ alkyl. The upper limit of the number of halogen substituents is equal to the total number of hydrogen atoms that can be substituted in the substitutable group. Without particular restriction under this limit, and the number of halogen substituents is any integer from 1 to the upper limit, preferably 1 to 5 halogen substituents, 1 to 3 halogen substituents, 1 to 2 halogen substituents, or 1 halogen substituent. When the number of halogen substituents is more than 1, they may be substituted by the same or different halogens. Non-limiting examples include monofluoromethoxy, difluoromethoxy, trifluoromethoxy, difluoroethoxy, and the like.

"Cycloalkyl" refers to a substituted or unsubstituted, saturated or partially unsaturated non-aromatic hydrocarbon ring. It can be monocyclic, bicyclic or polycyclic, and the bicyclic or polycyclic rings can be fused rings, spiro rings or bridged rings. Unless otherwise specified, it generally has 3 to 20 carbon atoms. When it is monocyclic cycloalkyl, it preferably has 3 to 15 carbon atoms, preferably 3 to 10 carbon atoms, more preferably 3 to 8 carbon atoms, more preferably 3 to 6 carbon atoms, and further preferably 3 to 4 carbon atoms. When it is bicyclic or polycyclic cycloalkyl, it preferably has 4 to 12 carbon atoms, preferably 4 to 11 carbon atoms, more preferably 5 to 11 carbon atoms, more preferably 6 to 11 carbon atoms, and further preferably 6 to 10 carbon atoms. Non-limiting examples include cyclopropyl, cyclobutyl, cyclopentyl, cyclohexyl, cycloheptyl, cyclobutenyl, cyclopentenyl, cyclohexenyl,

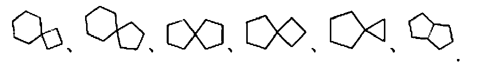

"Alkenyl" refers to a straight or branched hydrocarbon group containing at least one carbon-carbon double bond (C=C). Unless otherwise specified, it mainly contains 2 to 18 (e.g., 2 to 8, further e.g., 2 to 6, further e.g., 2 to 4) carbon atoms, including but not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 2-hexenyl. The alkenyl group may be optionally further substituted by any group.

"Heterocycloalkyl" refers to a substituted or unsubstituted, saturated or partially unsaturated non-aromatic ring containing at least one heteroatom. Unless otherwise specified, heterocycloalkyl is a 3-20 membered ring. When it is a monocyclic heterocycloalkyl, it is preferably 3-15 membered, preferably 3-10 membered, more preferably 3-8 membered, and further preferably 3-6 membered. When it is a bicyclic or polycyclic heterocycloalkyl, it is preferably 4-12 membered, preferably 4-11 membered, more preferably 5-11 membered, more preferably 6-11 membered, and further preferably 6-10 membered. Heterocycloalkyl may be monocyclic, bicyclic, or polycyclic. The ring can be a bridged ring, a fused ring, or a spiro ring, where the heteroatom is selected from N, S, O, P, or Si heteroatom and its oxidation state. When the heterocycloalkyl is bicyclic or polycyclic, at least one ring contains at least one heteroatom, and it can be a bicyclic or polycyclic ring formed by a heteroatom-containing ring and a non-heteroatom-containing ring; when connected to other groups, a heteroatom or a carbon atom may act as a connection point. Non-limiting examples include azetidinyl, morpholinyl, piperazinyl, piperidinyl, tetrahydropyranyl, oxetanyl, pyranyl, azacyclopentenyl, azacyclohexenyl, oxacyclopentenyl, and the like.

"Deuterium" refers to the isotope of hydrogen (H), i.e., deuterium.

As used herein, the term "cyano" refers to a group in which a carbon atom is triple-bonded to a nitrogen atom.

As used herein, the term "hydroxy" or "hydroxyl", whether used alone or in combination with other terms, means -OH.

The term "pharmaceutically acceptable salt" refers to a salt of the compound of the present invention that retains the biological efficacy and characteristics of a free acid or a free base, and is formed by mixing the free acid with a non-toxic inorganic or organic base, or the free base with a non-toxic inorganic or organic acid. This salt is a non-toxic salt formed by reaction with an inorganic or organic acid.

The term "pharmaceutical composition" refers to a mixture of one or more of the compounds described herein, or their stereoisomers, solvates, pharmaceutically acceptable salts, co-crystals, deuterated compounds, and other components, where the other components include physiologically/pharmaceutically acceptable carriers and/or excipients.

The term "stereoisomer" refers to an isomer resulting from different spatial arrangements of atoms in a molecule, including a cis-trans isomer, an enantiomer, and a conformational isomer.

The term "solvate" refers to a form formed by the combination of the compound of the present invention or a salt thereof with a stoichiometric or non-stoichiometric amount of solvent through intermolecular non-covalent bonds. When the solvent is water, it is a hydrate.

The term "co-crystal" refer to a crystal formed by the binding of an active pharmaceutical ingredient (API) and a co-crystal former (CCF) through hydrogen bonds or other non-covalent interactions, where both the API and CCF are solids in their pure states, and there is a fixed stoichiometric ratio between the components. The co-crystal is a multi-component crystal, including a binary co-crystal formed between two neutral solids and a multi-component co-crystal formed between a neutral solid and a salt or solvate.

The term "disease" refers to a physical state of the subject, which is related to the disease described in the present invention. For example, it is the disease related to Alzheimer's disease, bipolar disorder, Parkinson's disease, schizophrenia, diabetic peripheral neuropathic pain, postherpetic neuralgia, fibromyalgia, or peripheral neuropathy described in the present invention.

The term "carrier" refers to a system that does not cause significant irritation to organisms, does not eliminate the biological activity and properties of the administered compound, and can alter the way drugs enter the human body and drug distribution in the body, control the release rate of drugs, and deliver drugs to target organs. Non-limiting examples include liposomes, nanoparticles, and the like.

The compound or the pharmaceutical composition containing the same in the present invention can be administered in unit dosage form, and the route of administration can be enteral or parenteral, such as oral administration, intravenous injection, intramuscular injection, intravenous drip, subcutaneous injection, nasal administration, oral mucosa administration, ophthalmic administration, pulmonary and respiratory tract administration, cutaneous administration, vaginal administration, rectal administration, and the like.

The dosage forms for administration can be liquid dosage forms, solid dosage forms, or semi-solid dosage forms. The liquid dosage forms can be solutions (including true solutions and colloidal solutions), emulsions (including oil-in-water (o/w) type, water-in-oil (w/o) type, and multiple emulsions), suspensions, injections (including water injections, powder injections, and infusions), eye drops, nasal drops, lotions, liniments, and the like. The solid dosage forms can be tablets (including ordinary tablets, enteric-coated tablets, buccal tablets, dispersible tablets, chewable tablets, effervescent tablets, orally disintegrating tablets), capsules (including hard capsules, soft capsules, enteric-coated capsules), granules, powders, pellets, dropping pills, suppositories, films, patches, aerosols (dry powder inhalations), sprays, and the like. The semi-solid dosage forms can include ointments, gels, pastes, and the like.

To achieve the purpose of medication and enhance the therapeutic effect, the drug or pharmaceutical composition of the present invention can be administered by any known route of administration.

The compound or composition of the present invention can be taken alone or in combination with other therapeutic drugs or symptomatic drugs. When the compound of the present invention has a synergistic effect with other therapeutic drugs, its dosage should be adjusted according to the actual situation.

### Beneficial Technical Effects

The inventors have unexpectedly found that the compound in the present invention exhibits surprisingly good AKK1 inhibitory activity, with IC50 or EC50 comparable to or lower than that of the positive control drug, i.e. BMS-986176/LX-9211. The present invention provides a class of AKK1 inhibitor compounds with novel structures and strong activity. Such compounds have good application prospects in the prevention and/or treatment of indications related to AKK1 inhibition, such as Alzheimer's disease, bipolar disorder, Parkinson's disease, schizophrenia, diabetic peripheral neuropathic pain, postherpetic neuralgia, fibromyalgia, or peripheral neuropathy, and the like.

### BRIEF DESCRIPTION OF THE DRAWINGS

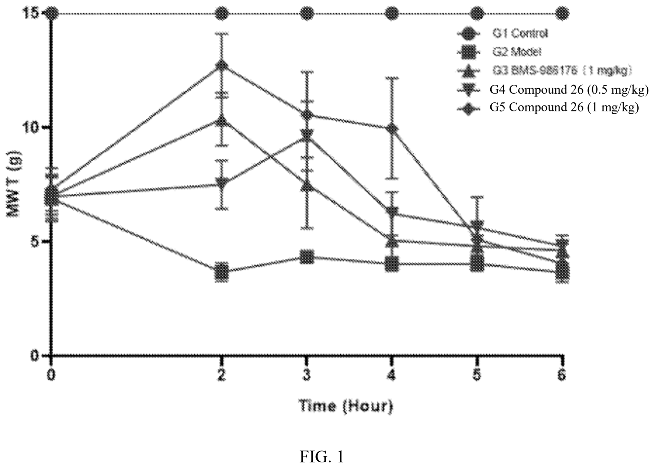

FIG. 1 shows an effect of single oral gavage (ig.) of Compound 26 on a pain threshold of peripheral neuropathic pain in diabetic rats.

### DESCRIPTION OF THE EMBODIMENTS

The examples listed below are helpful for those skilled in the art to better understand the technical solutions of the present invention, but do not limit the present invention in any way.

For all the following examples, standard operations and methods known to those skilled in the art can be used. Unless otherwise specified, all temperatures are expressed in °C (degrees Celsius). The structures of the compounds were determined by nuclear magnetic resonance spectroscopy (NMR) and/or mass spectrometry (MS).

The structures of the compounds of the present invention were determined by nuclear magnetic resonance (NMR) and/or liquid chromatography-mass spectrometry (LC-MS). NMR chemical shifts (δ) are given in parts per million (ppm). Nuclear magnetic resonance was measured using a Bruker avance-400 nuclear magnetic resonance spectrometer, with deuterated dimethyl sulfoxide (DMSO-d6), deuterated methanol (CD₃OD) and deuterated chloroform (CDCl₃) as solvents, and tetramethylsilane (TMS) as the internal standard.

For liquid chromatography-mass spectrometry (LC-MS) measurement, the liquid phase part was performed using an ACQUITY UPLC ultra-high pressure liquid chromatograph, and the mass spectrometry part was performed using an Xevo G2-S Qtof mass spectrometer.

The starting materials in the examples of the present invention are known and commercially available, or can be synthesized using or according to methods known in the art.

Those skilled in the art can prepare the compounds of the present invention by combining the literature of WO2017059085, WO2017059080 and WO2015153720, and known organic synthesis techniques. The starting materials are commercially available chemicals and/or compounds described in chemical literature. The "commercially available chemicals" were obtained from regular commercial channels, with suppliers including companies such as Shanghai Wuxi AppTec, Nanjing Chemical Industries, etc.

### Example 1: Synthesis of (R)-1-{[2-(difluoromethyl)-6-(pyrazolo[1,5-a]pyrimidin-7-yl)pyridin-3-yl]oxy}-2,4-dimet hylpentan-2-amine (Compound 1)

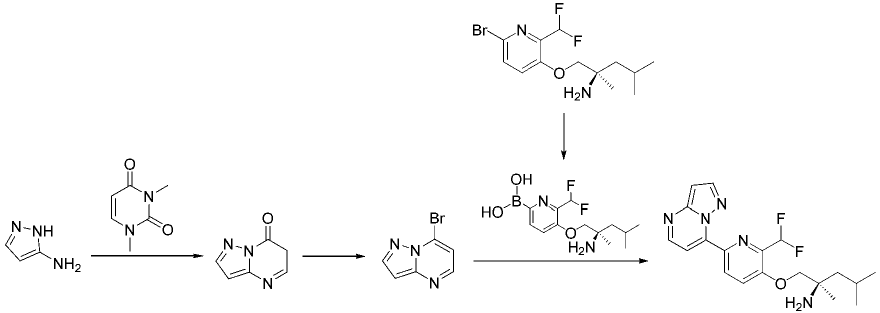

Step 1: 1H-pyrazol-5-amine (6.0 g, 72 mmol, 1.0 eq) and 1,3-dimethylpyrimidine-2,4(1H,3H)-dione (14.2 g, 100.8 mmol, 1.4 eq) were added to sodium ethoxide (60 mL, 20 wt%), and the mixture was reacted at 80°C for 3 h. After the reaction was completed, the mixture was cooled to 0°C. After filtration, the filter cake was dissolved in water, washed with dichloromethane three times, and the aqueous phase was adjusted to weakly acidic with acetic acid. The mixture was then cooled to 0°C to precipitate a solid. The solid was filtered and dried to obtain pyrazolo[1,5-a]pyrimidin-7(6H)-one as a white solid (5.8 g, 43.0 mmol, yield 59.7%). LCMS(TOF MS ES⁺)m/z [M+H]⁺: 136.04. ¹H NMR (400 MHz, DMSO) δ 12.08 (s, 1H), 8.47 (d, *J = 7.9* Hz, 1H), 7.75 (d, *J =* 1.9 Hz, 1H), 5.93 (d, *J =* 7.9 Hz, 1H), 5.81 (d, *J* = 2.0 Hz, 1H).

Step 2: Pyrazolo[1,5-a]pyrimidin-7(6H)-one (2.0 g, 14.8 mmol, 1.0 eq) and potassium carbonate (6.12 g, 44.4 mmol, 3.0 eq) were added to an acetonitrile solution, followed by the addition of phosphorus oxybromide (12.7 g, 44.40 mmol, 3.0 eq). After the addition, the mixture was reacted at 80°C for 5 h. After the reaction was completed, the reaction solution was poured into ice water, and a saturated sodium bicarbonate solution was added to neutralize phosphorus oxybromide. The aqueous phase was extracted with ethyl acetate three times, and the organic phases were combined and concentrated. Purification by normal-phase column separation (ethyl acetate/petroleum ether, ethyl acetate % = 25%) was performed to obtain 7-bromopyrazolo[1,5-a]pyrimidine as a yellow solid (800 mg, 4.06 mmol, yield 27.4%). LCMS(TOF MS ES⁺)m/z [M+H]⁺: 197.96. ¹H NMR (400 MHz, DMSO) δ 9.08 (d, *J = 7.1* Hz, 1H), 8.27 (d, *J =* 2.3 Hz, 1H), 7.22 (d, *J =* 7.2 Hz, 1H), 6.73 (d, *J =* 2.3 Hz, 1H).

Step 3: (R)-1-{[6-bromo-2-(difluoromethyl)pyridin-3-yl]oxy}-2,4-dimethylpentan-2-amine (200 mg, 0.59 mmol, 1.0 eq) and bis(pinacolato)diboron (299 mg, 1.18 mmol, 2.0 eq) were dissolved in 1,4-dioxane (12 mL), followed by the sequential addition of potassium acetate (173.7 mg, 1.77 mmol, 3.0 eq) and [1,1'-bis(diphenylphosphino)ferrocene]palladium dichloride (43.2 mg, 0.059 mmol, 0.1 eq). The mixture was reacted at 80°C for 16 hours under a nitrogen atmosphere. After the reaction was completed, the reaction solution was filtered. The filtrate was concentrated and purified by reverse-phase column separation (water/acetonitrile, acetonitrile % = 3-5%) to obtain (R)-{5-[(2-amino-2,4-dimethylpentyl)oxy]-6-(difluoromethyl)pyridin-2-yl} boronic acid as a white oil (35 mg, 0.11 mmol, yield 17.5%). LCMS(TOF MS ES⁺)m/z [M+H]⁺: 303.16. ¹H NMR (400 MHz, DMSO-*d*₆) δ 8.28 (d, *J =* 6.9 Hz, 2H), 7.90 - 7.53 (m, 2H), 7.27 (m, *J =* 53.7, 10.9 Hz, 1H), 3.95 (s, 2H), 1.79 (m*, J=* 11.6, 6.1 Hz, 1H), 1.57 - 1.40 (m, 2H), 1.31 (s, 3H), 0.92 (m, *J =* 14.7, 6.6, 3.4 Hz, 6H).

Step 4: (R)-{5-[(2-amino-2,4-dimethylpentyl)oxy]-6-(difluoromethyl)pyridin-2-yl}boronic acid (25 mg, 0.082 mmol, 1.0 eq) and 7-bromopyrazolo[1,5-a]pyrimidine (16.48 mg, 0.082 mmol, 1.0 eq) were dissolved in 2 mL of 1,4-dioxane, followed by the addition of sodium carbonate (35 mg, 0.33 mmol, 4.0 eq) and [1,1'-bis(diphenylphosphino)ferrocene]palladium dichloride (6.05 mg, 0.008 mmol, 0.1 eq). The system was purged with nitrogen twice and reacted at 120°C for 6 h. After the reaction was completed, purification by reverse-phase preparative chromatography was performed to obtain (R)-1-{[2-(difluoromethyl)-6-(pyrazolo[1,5-a]pyrimidin-7-yl)pyridin-3-yl]oxy}-2,4-dimethyl pentan-2-amine as a white solid (1.4 mg, 0.003 mmol, yield 5.6%). LCMS(TOF MS ES⁺)m/z [M+H]⁺: 376. ¹H NMR (400 MHz, Methanol-*d*₄) δ 8.99 - 8.91 (m, 1H), 8.67 (d, *J =* 8.8 Hz, 1H), 8.52 (s, 2H), 8.18 (d, *J=* 2.4 Hz, 1H), 8.06 (d, *J=* 7.4 Hz, 1H), 7.80 (d, *J=* 8.9 Hz, 1H), 7.26 - 6.95 (m, 1H), 6.74 (m, *J* = 2.3, 0.9 Hz, 1H), 4.24 - 4.09 (m, 2H), 1.85 (m, *J* = 12.6, 6.3 Hz, 1H), 1.75 (m, *J =* 14.3, 5.7 Hz, 1H), 1.63 (m, *J =* 14.2, 5.5 Hz, 1H), 1.41 (s, 3H), 1.02 (m, *J =* 14.9, 6.6 Hz, 6H).

### Example 2: Synthesis of (R)-1-{[2-(difluoromethyl)-6-(1H-pyrrolo[2,3-b]pyridin-3-yl)pyridin-3-yl]oxy}-2,4-dimet hylpentan-2-amine (Compound 2)

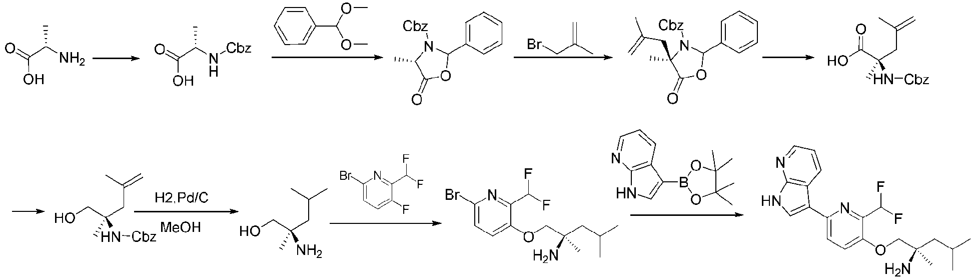

Step 1: D-alanine (43 g, 428.90 mmol, 1.0 eq) and sodium carbonate (153.55 g, 1448.70 mmol, 3.8 eq) were dissolved in purified water (540 mL). Benzyl chloroformate (93.90 g, 550.50 mmol, 1.3 eq) was slowly added dropwise at 0°C, and the mixture was reacted at room temperature for 16 hours. After the reaction was completed, water (1 L) and ethyl acetate (1 L) were added for extraction and liquid separation. The aqueous phase was adjusted to a pH value of 2 with 4 mol/L hydrochloric acid, and then ethyl acetate (1 L) was added for extraction and liquid separation. The organic phase was dried over anhydrous sodium sulfate and concentrated to obtain [(benzyloxy)carbonyl]-D-alanine as a white solid (55 g, yield 51.40%). LCMS(TOF MS ES⁺)m/z [M+H]⁺: 224.08. ¹H NMR (400 MHz, DMSO) δ 12.55 (s, 1H), 7.62 (d, *J =* 7.6 Hz, 1H), 7.41 - 7.24 (m, 5H), 5.02 (s, 2H), 4.05 - 3.99 (m, 1H), 1.26 *(d, J=* 7.3 Hz, 3H).

Step 2: The compounds [(benzyloxy)carbonyl]-D-alanine (55 g, 246.40 mmol, 1.0 eq) and benzaldehyde dimethyl acetal (45 g, 295.68 mmol, 1.2 eq) were dissolved in tetrahydrofuran (400 mL). Thionyl chloride (35.20 g, 295.68 mmol, 1.2 eq) was slowly added dropwise at 0°C. After reaction at 0°C for half an hour, zinc chloride (40.30 g, 295.68 mmol, 1.2 eq) was added, and the mixture was reacted at 0°C for 4 hours. After the reaction was completed, the reaction solution was diluted with 1000 mL of water and extracted three times with ethyl acetate (500 mL). The ethyl acetate phases were combined, and washed twice with water (800 mL) and once with saturated aqueous sodium chloride. The organic phase was dried over anhydrous sodium sulfate, filtered, then concentrated, and purified by normal-phase column chromatography (petroleum ether:ethyl acetate = 10:1) to obtain benzyl (4S)-4-methyl-5-oxo-2-phenyloxazolidine-3-carboxylate as a pale yellow oil (38 g, yield 49.54%). LCMS(TOF MS ES⁺)m/z [M+H]⁺: 312.12. ¹H NMR (400 MHz, DMSO) δ 7.53 - 7.41 (m, 5H), 7.41 - 7.18 (m, 5H), 6.58 (s, 1H), 5.06 (d, *J =* 26.7 Hz, 2H), 4.56 (q, *J =* 7.0 Hz, 1H), 1.52 (d, *J =* 7.0 Hz, 3H).

Step 3: Benzyl (4S)-4-methyl-5-oxo-2-phenyloxazolidine-3-carboxylate (38 g, 122.2 mmol, 1.0 eq) and 3-bromo-2-methyl-1-propene (20 g, 152.6 mmol, 1.25 eq) were dissolved in tetrahydrofuran. Under a nitrogen atmosphere, lithium bis(trimethylsilyl)amide (183.07 mL, 183.07 mmol, 1.5 eq) was added dropwise at -78°C, and the mixture was reacted for 3 hours. After the reaction was completed, a saturated aqueous ammonium chloride solution (15 mL) was added dropwise under an ice bath. Then, extraction and liquid separation were performed with ethyl acetate and water. The organic layer was collected, and washed twice with water and once with saturated brine. Purification by normal-phase column separation was performed, and when the ethyl acetate content was 6%, benzyl (4R)-4-methyl-4-(2-methylallyl)-5-oxo-2-phenyloxazolidine-3-carboxylate was obtained as a transparent oil (19 g, yield 43.20%). LCMS(TOF MS ES⁺)m/z [M+H]⁺: 366.16. ¹H NMR (400 MHz, DMSO) δ 7.45 (m, *J =* 11.4, 5.9 Hz, 7H), 7.19 (m, *J =* 14.6, 7.2 Hz, 2H), 6.79 (d, *J* = 7.3 Hz, 1H), 6.45 (d, *J =* 26.5 Hz, 1H), 5.21 - 5.07 (m, 1H), 5.03 - 4.85 (m, 2H), 4.74 - 4.54 (m, 1H), 3.20 - 2.85 (m, 1H), 2.42 (m, *J =* 13.8, 8.9 Hz, 1H), 1.82 - 1.56 (m, 6H).

Step 4: Benzyl (4R)-4-methyl-4-(2-methylallyl)-5-oxo-2-phenyloxazolidine-3-carboxylate (19 g, 52 mmol, 1.0 eq) was dissolved in tetrahydrofuran. Then, a lithium hydroxide hydrate (2.4 g, 57.20 mmol, 1.1 eq) was dissolved in water and added dropwise to the reaction system at 0°C. Subsequently, the mixture was allowed to react at room temperature for 16 hours. After the reaction was completed, the mixture was extracted with ethyl acetate and water. The aqueous phase was adjusted to a pH value of 5-6 and subjected to extraction and liquid separation again with ethyl acetate. The organic phase was concentrated to obtain (R)-2-[(benzyloxycarbonyl)amino]-2,4-dimethylpent-4-enoic acid as a transparent oil (9.0 g, yield 42.10%). LCMS(TOF MS ES⁺)m/z [M+H]⁺: 278. ¹H NMR (400 MHz, DMSO) δ 12.45 (s, 1H), 7.35 - 7.17 (m, 5H), 4.96 (d, *J=* 3.7 Hz, 2H), 4.78 (dd, *J =* 2.7, 1.5 Hz, 1H), 4.63 (d, *J =* 2.5 Hz, 1H), 2.63 - 2.36 (m, 2H), 1.61 (s, 3H), 1.26 (s, 3H).

Step 5: (R)-2-[(benzyloxycarbonyl)amino]-2,4-dimethylpent-4-enoic acid (9.0 g, 32.5 mmol, 1.0 eq) and triethylamine (4.9 g, 48.75 mmol, 1.5 eq) were dissolved in THF (30 mL). Under a nitrogen atmosphere, isobutyl chloroformate (5.3 g, 39 mmol, 1.2 eq) was added dropwise to the system at -15°C. After reaction at this temperature for 0.5 h, the mixture was filtered, and the filtrate was retained. An aqueous solution of sodium borohydride was added to the filtrate at 0°C, and the mixture was reacted at room temperature for 10 minutes. After the reaction was completed, water was added to quench the sodium borohydride. The mixture was subjected to extraction and liquid separation with ethyl acetate and water. The organic phase was concentrated and purified by normal-phase column separation. The target product was eluted when the ethyl acetate content was 30%, and concentrated to obtain benzyl (R)-(1-hydroxy-2,4-dimethylpent-4-en-2-yl)carbamate as a transparent oil (3.8 g, yield 44.46%). LCMS(TOF MS ES⁺)m/z [M+H]⁺:264/265. ¹H NMR (400 MHz, DMSO) δ 7.42 - 7.25 (m, 5H), 6.73 (s, 1H), 5.07 - 4.91 (m, 2H), 4.79 (m, *J =* 3.0, 1.6 Hz, 1H), 4.71 (t, *J =* 5.7 Hz, 1H), 4.64 (d, *J =* 2.7 Hz, 1H), 3.46 - 3.32 (m, 2H), 2.47 (s, 1H), 2.21 (d, *J =* 13.2 Hz, 1H), 1.68 (s, 3H), 1.10 (s, 3H).

Step 6: The compound benzyl (R)-(1-hydroxy-2,4-dimethylpent-4-en-2-yl)carbamate (1.5 g, 5.70 mmol) was dissolved in methanol (20.0 mL). Palladium on carbon (150 mg, 10%) was added, and the mixture was reacted under a hydrogen atmosphere at room temperature for 16 hours. After the reaction was completed, the mixture was filtered by adding diatomaceous earth. The filtrate was concentrated, and then water (100 mL) and ethyl acetate (100 mL) were added for extraction and liquid separation. The organic phase was separated, then washed twice with water (100 mL) and once with a saturated sodium chloride solution (100 mL), finally dried over anhydrous sodium sulfate, and concentrated to obtain (R)-2-amino-2,4-dimethylpentan-1-ol as a white solid product (620 mg, yield 82.90%). LCMS(TOF MS ES⁺)m/z [M+H]⁺: 132.

Step 7: The compound (R)-2-amino-2,4-dimethylpentan-1-ol (80 mg, 4.42 mmol, 1.0 eq) was added to tetrahydrofuran (30.0 mL), followed by the addition of potassium tert-butoxide (1.24 g, 11.05 mmol, 2.5 eq) and 6-bromo-2-(difluoromethyl)-3-fluoropyridine (1.49 g, 6.63 mmol, 1.5 eq). The mixture was reacted at 93°C for 2 hours. After the reaction was completed, the mixture was filtered, and the filtrate was concentrated and purified by normal-phase column chromatography to obtain (R)-1-{[6-bromo-2-(difluoromethyl)pyridin-3-yl]oxy}-2,4-dimethylpentan-2-amine as a brown oily product (926 mg, yield 62.11%). LCMS(TOF MS ES⁺)m/z [M+H]⁺: 337/339. ¹H NMR (400 MHz, DMSO) δ 7.79 (d, *J =* 8.8 Hz, 1H), 7.66 (d, *J =* 8.9 Hz, 1H), 7.16 (t, *J =* 53.4 Hz, 1H), 3.81 (s, 2H), 1.79 (m, *J =* 12.7, 6.3 Hz, 1H), 1.68 (s, 2H), 1.10 (s, 3H), 0.92 (m, *J* = 8.4, 6.6 Hz, 6H).

Step 8: (R)-1-{[6-bromo-2-(difluoromethyl)pyridin-3-yl]oxy}-2,4-dimethylpentan-2-amine (40 mg, 0.12 mmol, 1.0 eq), 7-azaindole-3-boronic acid pinacol ester (28.95 mg, 0.12 mmol, 1.0 eq), sodium carbonate (50 mg, 0.48 mmol, 4.0 eq), and [1,1'-bis(diphenylphosphino)ferrocene]palladium dichloride (8.78 mg, 0.12 mmol, 1.0 eq) were added to 1,4-dioxane (3.0 mL). The mixture was reacted at 120°C for 6 hours under a nitrogen atmosphere. After the reaction was completed, the mixture was filtered, and the filtrate was purified by reverse-phase system on a C18 column to obtain (R)-1-{[2-(difluoromethyl)-6-(1H-pyrrolo[2,3-b]pyridin-3-yl)pyridin-3-yl]oxy}-2,4-dimethyl pentan-2-amine as a white solid (8.60 mg, yield 19.32%). LCMS(TOF MS ES⁺)m/z [M+H]⁺: 375.2. ¹H NMR (400 MHz, MeOD) δ 8.95 (m, *J =* 8.0, 1.7 Hz, 1H), 8.55 (s, 1H), 8.29 (d, *J =* 4.7 Hz, 1H), 8.06 (d, *J =* 1.7 Hz, 1H), 7.99 (d, *J =* 8.8 Hz, 1H), 7.69 (d, *J =* 8.9 Hz, 1H), 7.34 - 7.03 (m, 2H), 4.29 - 4.23 (m, 1H), 4.21 - 4.14 (m, 1H), 1.95 - 1.83 (m, 2H), 1.77 - 1.67 (m, 1H), 1.53 (d, *J =* 1.7 Hz, 3H), 1.08 (m, *J =* 15.5, 6.5, 1.8 Hz, 6H).

### Example 16: Synthesis of (S)-1-{[2-(difluoromethyl)-6-(4-methoxy-1H-pyrrolo[2,3-b]pyridin-3-yl)pyridin-3-yl]oxy }-2,4-dimethylpentan-2-amine tris(trifluoroacetate) (Compound 16)

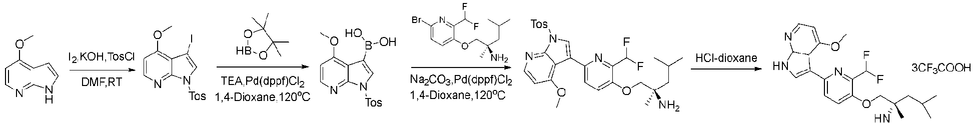

### Step 1: Synthesis of 3-iodo-4-methoxy-1-tosyl-1H-pyrrolo[2,3-b]pyridine

Compounds 4-methoxy-7-azaindole (200 mg, 1.35 mmol, 1.0 eq) and potassium hydroxide (189 mg, 3.37 mmol, 2.5 eq) were added to N,N-dimethylformamide (3.0 mL). Iodine (360 mg, 1.42 mmol, 1.05 eq) was dissolved in N,N-dimethylformamide (2.0 mL), and the resulting solution was slowly added to the above system. The mixture was reacted at room temperature for 45 minutes. Then, additional potassium hydroxide (189 mg, 3.37 mmol, 2.5 eq) was added. After reaction for 10 minutes, p-toluenesulfonyl chloride (540 mg, 2.83 mmol, 2.1 eq) was added, and the mixture was reacted at room temperature for 2.5 hours. After the reaction was completed, the reaction mixture was poured into water (20 mL), and a solid precipitated out and was filtered. The filter cake was washed with water (10 mL) and ethyl acetate (20 mL) separately. Then, the filter cake was collected and dried to obtain 3-iodo-4-methoxy-1-tosyl-1H-pyrrolo[2,3-b]pyridine as a yellow solid (360 mg, yield 62.28%). LCMS(TOF MS ES+)m/z [M+H]+: 428.9760. ¹H NMR (400 MHz, DMSO) δ 8.29 (d, *J =* 5.7 Hz, 1H), 8.04 - 7.98 (m, 2H), 7.90 (s, 1H), 7.44 (d, *J =* 8.1 Hz, 2H), 6.94 (d, *J* = 5.7 Hz, 1H), 3.94 (s, 3H), 2.36 (s, 3H).

### Step 2: Synthesis of (4-methoxy-1-tosyl-1H-pyrrolo[2,3-b]pyridin-3-yl)boronic acid

3-Iodo-4-methoxy-1-tosyl-1H-pyrrolo[2,3-b]pyridine (310 mg, 0.72 mmol, 1.0 eq), pinacolborane (463 mg, 3.62 mmol, 5.0 eq), triethylamine (366 mg, 3.62 mmol, 5.0 eq), and [1,1'-bis(diphenylphosphino)ferrocene]palladium dichloride (52 mg, 0.072 mmol, 0.10 eq) were added to 1,4-dioxane (3.0 mL). The mixture was reacted at 120°C for 2 hours under a nitrogen atmosphere. After the reaction was completed, the mixture was filtered. The filtrate was concentrated and purified by reverse-phase chromatography on a C18 column to obtain (4-methoxy-1-tosyl-1H-pyrrolo[2,3-b]pyridin-3-yl)boronic acid as a yellow solid (150 mg, yield 59.86%). LCMS(TOF MS ES+)m/z [M+H]+: 347.1177. ¹H NMR (400 MHz, DMSO) δ 8.28 (d, J = 5.7 Hz, 1H), 8.04 - 7.98 (m, 3H), 7.86 (s, 2H), 7.43 (d, J = 8.1 Hz, 2H), 6.99 (d, J = 5.7 Hz, 1H), 4.02 (s, 3H).

### Step 3: Synthesis of (S)-1-{[2-(difluoromethyl)-6-(4-methoxy-1-tosyl-1H-pyrrolo[2,3-b]pyridin-3-yl)pyridin-3 -yl]oxy}-2,4-dimethylpentan-2-amine

(4-Methoxy-1-tosyl-1H-pyrrolo[2,3-b]pyridin-3-yl)boronic acid (100 mg, 0.29 mmol, 1.0 eq), (S)-1-{[6-bromo-2-(difluoromethyl)pyridin-3-yl]oxy}-2,4-dimethylpentan-2-amine (97.41 mg, 0.29 mmol, 1.0 eq), sodium carbonate (122.50 mg, 1.06 mmol, 4.0 eq), and [1,1'-bis(diphenylphosphino)ferrocene]palladium dichloride (21.14 mg, 0.029 mmol, 0.10 eq) were added to 1,4-dioxane (3.0 mL). The mixture was reacted at 120°C for 8 hours under a nitrogen atmosphere. After the reaction was completed, the mixture was filtered. The filtrate was concentrated and purified by normal-phase column chromatography (dichloromethane:methanol = 12:1) to obtain (S)-1-{[2-(difluoromethyl)-6-(4-methoxy-1-tosyl-1H-pyrrolo[2,3-b]pyridin-3-yl)pyridin-3-yl] oxy}-2,4-dimethylpentan-2-amine as a brown oily product (150 mg, yield 92.95%). LCMS(TOF MS ES+)m/z [M+H]+: 559.2274. ¹H NMR (400 MHz, DMSO) δ 8.37 - 8.29 (m, 1H), 8.07 (d, *J =* 8.0 Hz, 2H), 8.03 - 7.97 (m, 2H), 7.70 (d, *J =* 8.9 Hz, 1H), 7.45 (d, *J =* 8.0 Hz, 2H), 7.38 - 7.08 (m, 1H), 7.04 - 6.97 (m, 1H), 4.12 - 3.98 (m, 2H), 3.91 (d, *J =* 2.2 Hz, 3H), 2.37 (s, 3H), 2.00 (d, *J =* 2.2 Hz, 2H), 1.81 (d, *J =* 7.5 Hz, 1H), 1.18 - 1.16 (m, 3H), 0.95 (ddd, *J = 9.0,* 6.9, 2.1 Hz, 6H).

### Step 4: Synthesis of (S)-1-{[2-(difluoromethyl)-6-(4-methoxy-1H-pyrrolo[2,3-b]pyridin-3-yl)pyridin-3-yl]oxy }-2,4-dimethylpentan-2-amine tris(trifluoroacetate)

(S)-1-{[2-(difluoromethyl)-6-(4-methoxy-1-tosyl-1H-pyrrolo[2,3-b]pyridin-3-yl)pyridin-3-yl]oxy}-2,4-dimethylpentan-2-amine (150 mg, 0.27 mmol, 1.0 eq) was added to a solution of hydrogen chloride in dioxane (5.0 mL, 4.0 mol/L). The mixture was reacted at 60°C for 16 hours. After the reaction was completed, the mixture was directly filtered. The filter cake was washed with a mixed solvent (petroleum ether:ethyl acetate = 5:1, 20 mL) to remove excess impurities. The collected filter cake was purified by reverse-phase preparative chromatography on a C18 column (formic acid system) to obtain (S)-1-{[2-(difluoromethyl)-6-(4-methoxy-1H-pyrrolo[2,3-b]pyridin-3-yl)pyridin-3-yl]oxy}-2, 4-dimethylpentan-2-amine tris(trifluoroacetate) as a white solid (6.2 mg, yield 4.26%). LCMS(TOF MS ES+)m/z [M+H]+: 391.2328. ¹H NMR (400 MHz, DMSO) δ 12.12 (s, 1H), 8.23 (d, *J* = 5.6 Hz, 1H), 8.07 (d, *J =* 8.9 Hz, 1H), 7.77 - 7.71 (m, 2H), 7.61 - 7.30 (m, 1H), 6.85 (d, *J* = 5.7 Hz, 1H), 4.25 - 4.11 (m, 2H), 3.98 (s, 3H), 1.89 - 1.71 (m, 2H), 1.61 (dd, *J =* 14.1, 5.3 Hz, 1H), 1.40 (s, 3H), 1.04 - 0.90 (m, 6H).

### Example 17: Synthesis of (R)-1-{[2-(difluoromethyl)-6-(4-methoxy-1H-pyrrolo[2,3-b]pyridin-3-yl)pyridin-3-yl]oxy }-2,4-dimethylpentan-2-amine (Compound 17)

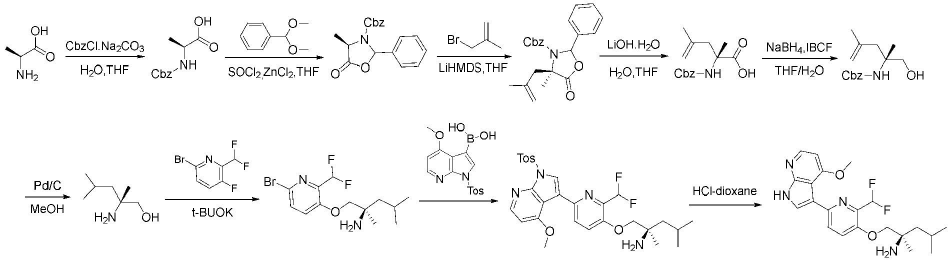

### Step 1: Synthesis of [(benzyloxy)carbonyl]-L-alanine

L-alanine (50 g, 561.15 mmol, 1.0 eq) and sodium carbonate (118.72 g, 1122.96 mmol, 2.0 eq) were dissolved in water (540 mL). Then, benzyl chloroformate (105.36 g, 550.50 mmol, 1.10 eq) was added to 1,4-dioxane (83 mL). The benzyl chloroformate (105.36 g, 550.50 mmol, 1.10 eq) solution was slowly added dropwise at 0°C. After reaction at 0°C for 0.5 hours, the mixture was allowed to react at room temperature for 16 hours. After the reaction was completed, water (500 mL) and ethyl acetate (750 mL) were added for two extractions and liquid separations. The aqueous phase was adjusted to a pH value of 2-4 with 4 mol/L hydrochloric acid, and then ethyl acetate (1 L) was added for extraction and liquid separation. The organic phase was dried over anhydrous sodium sulfate and concentrated to obtain [(benzyloxy)carbonyl]-L-alanine as a white solid (101.00 g, yield 80.62%). ¹H NMR (400 MHz, DMSO) *δ* 12.52 (s, 1H), 7.60 (d, *J =* 7.6 Hz, 1H), 7.44 - 7.16 (m, 65H), 5.02 (s, 2H), 4.07 - 3.94 (m, 1H), 1.28 - 1.13 (m, 3H).

### Step 2: Synthesis of benzyl (4R)-4-methyl-5-oxo-2-phenyloxazolidine-3-carboxylate

The compounds [(benzyloxy)carbonyl]-L-alanine (101 g, 452.71 mmol, 1.0 eq) and benzaldehyde dimethyl acetal (72.29 g, 475.37 mmol, 1.05 eq) were dissolved in tetrahydrofuran (500 mL). Thionyl chloride (64.62 g, 543.24 mmol, 1.2 eq) was slowly added at 0°C. After reaction for half an hour, zinc chloride (74.04 g, 543.26 mmol, 1.2 eq) was added, and the reaction was continued at 0°C for 4 hours. After the reaction was completed, the reaction solution was diluted with water (1 L) and extracted three times with ethyl acetate (500 mL). The ethyl acetate phases were combined and washed twice with water (800 mL) and once with saturated aqueous sodium chloride. The organic phase was dried over anhydrous sodium sulfate, concentrated, and purified by normal-phase column chromatography to obtain benzyl (4R)-4-methyl-5-oxo-2-phenyloxazolidine-3-carboxylate as a pale yellow oil (80.7 g, yield 57.29%). ¹H NMR (400 MHz, DMSO) δ 7.49 - 7.36 (m, 6H), 7.36 - 7.24 (m, 4H), 6.54 (s, 1H), 5.16 - 4.84 (m, 2H), 4.52 (q, J = 7.0 Hz, 1H), 1.48 (d, *J=* 7.0 Hz, 3H).

### Step 3: Synthesis of benzyl (4R)-4-methyl-4-(2-methylallyl)-5-oxo-2-phenyloxazolidine-3-carboxylate

Benzyl (4R)-4-methyl-5-oxo-2-phenyloxazolidine-3-carboxylate (80.7 g, 259.5 mmol, 1.0 eq) and 3-bromo-2-methyl-1-propene (42.5 g, 324.1 mmol, 1.25 eq) were dissolved in tetrahydrofuran (500 mL). Under a nitrogen atmosphere, lithium bis(trimethylsilyl)amide (388 mL, 389.1 mmol, 1.5 eq) was added dropwise at -78°C over 15 minutes, and the mixture was reacted for 3 hours. After the reaction was completed, a saturated aqueous ammonium chloride solution (25 mL) was added dropwise under an ice bath. Then, extraction and liquid separation were performed with ethyl acetate and water. The organic layer was collected, and washed twice with water and once with saturated brine. Purification by normal-phase column separation was performed, when the ethyl acetate content was 6%, the product eluted, and then concentrated to obtain benzyl (4R)-4-methyl-4-(2-methylallyl)-5-oxo-2-phenyloxazolidine-3-carboxylate as a transparent oil (40.76 g, yield 43.03%). LCMS(ESI)[M+H]+: 366. ¹H NMR (400 MHz, DMSO) *δ* 7.47 - 7.32 (m, 7H), 7.16 (m, *J =* 14.6, 7.2 Hz, 2H), 6.76 (d, *J =* 7.3 Hz, 1H), 6.49 - 6.33 (m, 1H), 5.16 - 5.04 (m, 1H), 4.98 - 4.83 (m, 2H), 4.70 - 4.51 (m, 1H), 3.16 - 2.78 (m, 1H), 2.39 (dd, *J =* 13.8, 8.5 Hz, 1H), 1.76 - 1.50 (m, 6H).

### Step 4: Synthesis of (R)-2-[(benzyloxycarbonyl)amino]-2,4-dimethylpent-4-enoic acid

Benzyl (4R)-4-methyl-4-(2-methylallyl)-5-oxo-2-phenyloxazolidine-3-carboxylate (16.72 g, 45.8 mmol, 1.0 eq) was dissolved in tetrahydrofuran (100 mL). Then, a lithium hydroxide hydrate (2.88 g, 68.67 mmol, 1.15 eq) was dissolved in water (100 mL) and added dropwise to the reaction system at 0°C. Subsequently, the mixture was allowed to react at room temperature for 16 hours. After the reaction was completed, the mixture was extracted with ethyl acetate and water. The aqueous phase was adjusted to a pH value of 5-6 and subjected to three extractions and liquid separations again with ethyl acetate. The organic phase was concentrated to obtain (R)-2-[(benzyloxycarbonyl)amino]-2,4-dimethylpent-4-enoic acid as a transparent oil (6.84 g, yield 53.91%). LCMS(ESI)[M+H]+: 278. ¹H NMR (400 MHz, DMSO) 612.59 (s, 1H), 7.35 - 7.19 (m, 5H), 4.95 (s, 2H), 4.75 (dd, *J* = 2.7, 1.5 Hz, 1H), 4.63 - 4.58 (m, 1H), 2.56 (d, *J =* 13.4 Hz, 1H), 2.44 - 2.40 (m, 1H), 1.59 (s, 3H), 1.26 (s, 3H).

### Step 5: Synthesis of (R)-(1-hydroxy-2,4-dimethylpent-4-en-2-yl)carbamate

(R)-2-[(benzyloxy)carbonyl]amino-2,4-dimethylpent-4-enoic acid (6.8 g, 24.5 mmol, 1.0 eq) and triethylamine (3.71 g, 36.7 mmol, 1.5 eq) were dissolved in tetrahydrofuran (25 mL). Under a nitrogen atmosphere, isobutyl chloroformate (4.02 g, 24.3 mmol, 1.2 eq) was added dropwise to the system at -15°C. After reaction at this temperature for 0.5 h, the mixture was filtered, and the filtrate was retained. A solution of sodium borohydride (3.7 g, 98.08 mmol, 4.0 eq) in water (15 mL) was added to the filtrate at 0°C, and the mixture was reacted at room temperature for 10 minutes. After the reaction was completed, water was added to quench the sodium borohydride. The mixture was subjected to extraction and liquid separation with ethyl acetate and water. The organic phase was concentrated and purified by normal-phase column separation. The target product was eluted when the ethyl acetate content was 23%, and concentrated to obtain benzyl (R)-(1-hydroxy-2,4-dimethylpent-4-en-2-yl)carbamate as a transparent oil (4.26 g, yield 66.01%). LCMS(ESI)[M+H]+: 264. ¹H NMR (400 MHz, DMSO) *δ* 7.37 - 7.22 (m, 5H), 6.66 (s, 1H), 5.01 - 4.88 (m, 2H), 4.76 (m, *J =* 3.0, 1.5 Hz, 1H), 4.70 - 4.58 (m, 2H), 3.43 - 3.31 (m, 2H), 2.44 (s, 1H), 2.18 (d, *J =* 13.2 Hz, 1H), 1.65 (s, 3H), 1.08 (s, 3H).

### Step 6: Synthesis of (R)-2-amino-2,4-dimethylpentan-1-ol

Benzyl (R)-(1-hydroxy-2,4-dimethylpent-4-en-2-yl)carbamate (2.0 g, 7.6 mmol, 1.0 eq) was added to methanol (25 mL), followed by the addition of Pd/C (0.2 g, 10% wt) under a hydrogen atmosphere. The mixture was reacted at room temperature for 14 hours. After the reaction was completed, the mixture was filtered, and the filtrate was concentrated to obtain (R)-2-amino-2,4-dimethylpentan-1-ol as a yellow oil (1.06 g, crude). The product was used directly in the next step without further purification. ¹H NMR (400 MHz, DMSO) *δ* 3.07 (s, 2H), 1.79 - 1.67 (m, 1H), 1.28 - 1.08 (m, 2H), 0.97 - 0.74 (m, 9H).

### Step 7: Synthesis of (R)-1-{[6-bromo-2-(difluoromethyl)pyridin-3-yl]oxy}-2,4-dimethylpentan-2-amine

The compound (R)-2-amino-2,4-dimethylpentan-1-ol (200 mg, crude) was added to tetrahydrofuran (4 mL), followed by the addition of potassium tert-butoxide (4.0 g, 11.05 mmol, 2.5 eq) and 6-bromo-2-(difluoromethyl)-3-fluoropyridine (350 mg, 1.55 mmol, 1.0 eq). The mixture was reacted at 80°C for 3 hours. After the reaction was completed, the mixture was filtered, and the filtrate was concentrated to obtain (R)-1-1[6-bromo-2-(difluoromethyl)pyridin-3-yl]oxyl-2,4-dimethylpentan-2-amine as a brown oily liquid (255 mg, crude). The product was used directly in the next step without further purification. LCMS(TOF MS ES+)m/z [M+H]⁺: 337. ¹H NMR (400 MHz, DMSO) *δ* 7.75 (d, *J =* 8.8 Hz, 1H), 7.63 (d, *J =* 8.8 Hz, 1H), 7.38 - 6.78 (m, 1H), 3.80 (s, 2H), 2.02 - 1.59 (m, 2H), 1.48 - 1.26 (m, 2H), 1.21 (d, *J =* 8.7 Hz, 1H), 1.08 (s, 3H), 0.89 (dd, *J =* 8.7, 6.6 Hz, 6H).

### Step 8: Synthesis of (R)-1-{[2-(difluoromethyl)-6-(4-methoxy-1-tosyl-1H-pyrrolo[2,3-b]pyridin-3-yl)pyridin-3-yl]oxy}-2,4-dimethylpentan-2-amine

(R)-1-{[6-bromo-2-(difluoromethyl)pyridin-3-yl]oxy}-2,4-dimethylpentan-2-a mine (255 mg, crude), (4-methoxy-1-tosyl-1H-pyrrolo[2,3-b]pyridin-3-yl)boronic acid (261 mg, 0.756 mmol, 1.0 eq), sodium carbonate (320.58 mg, 3.02 mmol, 4.0 eq), and [1,1'-bis(diphenylphosphino)ferrocene]palladium dichloride (55.4 mg, 0.0756 mmol, 0.1 eq) were added to 1,4-dioxane (10 mL). The mixture was reacted at 120°C for 12 hours under a nitrogen atmosphere. After the reaction was completed, the mixture was filtered. The filtrate was concentrated and purified by normal-phase column chromatography (dichloromethane:methanol = 19:1) to obtain (R)-1-{[2-(difluoromethyl)-6-(4-methoxy-1-tosyl-1H-pyrrolo[2,3-b]pyridin-3-yl)pyridin-3-yl ]oxy}-2,4-dimethylpentan-2-amine as a brown solid product (364 mg, yield 86.23%). LCMS(TOF MS ES+)m/z [M+H]+: 559. 1H NMR (400 MHz, DMSO) δ 8.27 (d, J = 5.6 Hz, 1H), 8.13 - 7.96 (m, 3H), 7.93 (d, J = 10.9 Hz, 3H), 7.64 (d, J = 8.9 Hz, 1H), 7.39 (d, J = 8.0 Hz, 2H), 7.29 - 6.98 (m, 1H), 6.95 (d, J = 5.7 Hz, 1H), 3.87 (s, 3H), 3.81 (s, 2H), 2.32 (s, 3H), 1.42 - 1.31 (m, 2H), 1.19 (d, J = 5.5 Hz, 1H), 1.10 (s, 3H), 0.89 (t, J = 7.1 Hz, 6H).

### Step 9: Synthesis of (R)-1-{[2-(difluoromethyl)-6-(4-methoxy-1H-pyrrolo[2,3-b]pyridin-3-yl)pyridin-3-yl]oxy }-2,4-dimethylpentan-2-amine

(R)-1-{[2-(difluoromethyl)-6-(4-methoxy-1-tosyl-1H-pyrrolo[2,3-b]pyridin-3-yl)pyridin-3-yl]oxy}-2,4-dimethylpentan-2-amine (177 mg, 0.44 mmol, 1.0 eq) was added to a solution of hydrogen chloride in dioxane (3.5 mL, 4.0 mol/L). The mixture was reacted at 60°C for 1 hour and then at room temperature for 16 hours. After the reaction was completed, an insoluble solid precipitated. The mixture was filtered, and the filtrate was concentrated. The solid and filtrate were dissolved in methanol (3 mL) to obtain (R)-1-{[2-(difluoromethyl)-6-(4-methoxy-1H-pyrrolo[2,3-b]pyridin-3-yl)pyridin-3-yl]oxy}-2 ,4-dimethylpentan-2-amine as a white solid (16.1 mg, yield 12.56%). LCMS(TOF MS ES+)m/z [M+H]⁺: 405.2290. ¹H NMR (400 MHz, DMSO)*δ* 11.91 (s, 1H), 8.13 (d, *J =* 5.6 Hz, 1H), 7.99 (d, *J =* 8.8 Hz, 1H), 7.67 - 7.56 (m, 2H), 7.29 - 6.87 (m, 1H), 6.73 (d, *J =* 5.7 Hz, 1H), 3.91 (s, 3H), 3.77 (s, 2H), 1.78 (m, *J =* 6.4 Hz, 1H), 1.41 - 1.30 (m, 2H), 1.09 (s, 3H), 0.90 (t, *J =* 6.7 Hz, 6H).

### Example 26: Synthesis of (S)-1-((2-(difluoromethyl)-6-(5-fluoro-1H-pyrrolo[2,3-b]pyridin-3-yl)pyridin-3-yl)oxy)-2, 4-dimethylpentan-2-amine(Compound 26)

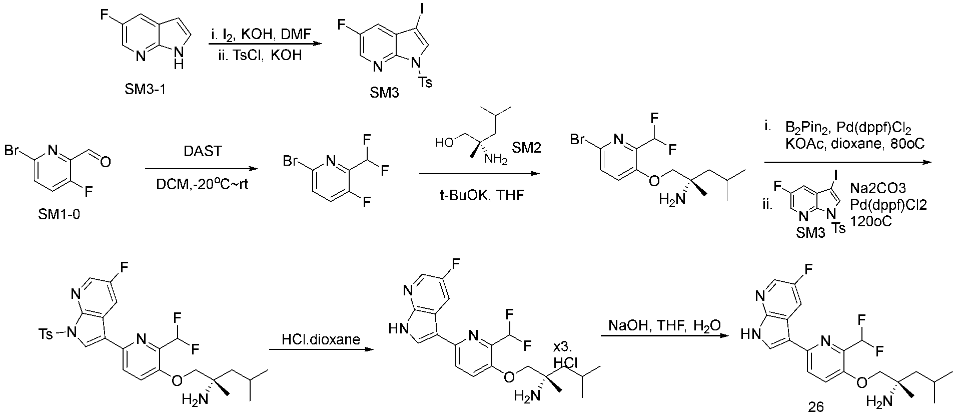

Step 1: 5-Fluoro-1H-pyrrolo[2,3-b]pyridine (600 mg, 4.4 mmol, 1.0 eq) and potassium hydroxide (616.0 mg, 11.0 mmol, 2.5 eq) were added to N,N-dimethylformamide (5 mL). A solution of iodine (586.7 mg, 4.62 mmol, 1.05 eq) dissolved in N,N-dimethylformamide (5 mL) was slowly added to the system, and the mixture was reacted for 45 minutes. Potassium hydroxide (616.0 mg, 11.0 mmol, 2.5 eq) was supplemented, and the reaction was continued for 5 minutes. Subsequently, a solution of p-toluenesulfonyl chloride (1.76 g, 9.24 mmol, 2.1 eq) dissolved in N,N-dimethylformamide (3 mL) was slowly added dropwise to the system, and the mixture was reacted at room temperature for 3 hours. After the reaction was completed, the system was poured into water (50 mL), stirred for 10 minutes, and then directly filtered. The filter cake was washed with water (20 mL) and ethyl acetate (20 mL), and dried to obtain 5-fluoro-3-iodo-1-p-tosyl-1H-pyrrolo[2,3-b]pyridine as a yellow solid product (957 mg, yield 69.75%).

Step 2: 6-Bromo-3-fluoropyridinecarbaldehyde (20 g, 98.04 mmol, 1.0 eq) was dissolved in anhydrous dichloromethane (200 mL). Diethylaminosulfur trifluoride (DAST, 27.2 mL, 196.08 mmol, 2.0 eq) was added dropwise to the system at -20°C, and the mixture was reacted at room temperature for 4 hours. After the reaction was completed, the system was poured into cold saturated aqueous sodium bicarbonate solution (200 mL) for quenching. Dichloromethane (200 mL) was added for extraction and liquid separation, and the organic phase was collected, washed twice with water (250 mL), dried, and concentrated to obtain a red solid. Then, silica gel was added to a suction funnel for decolorization to obtain 6-bromo-2-(difluoromethyl)-3-fluoropyridine as white crystals (19.0 g, yield 85.75%). LCMS(TOF MS ES+)m/z [M+H]+: 225.94. ¹H NMR (400 MHz, DMSO) δ 7.97 (d, *J =* 6.7 Hz, 2H), 7.33-6.78 (m, 1H).

Step 3: A compound (S)-2-amino-2,4-dimethylpentan-1-ol (17.74 g, 135 mmol, 1 eq) was added to tetrahydrofuran (450 mL), followed by the addition of potassium tert-butoxide (37.87 g, 337.5 mmol, 2.5 eq) and 6-bromo-2-(difluoromethyl)-3-fluoropyridine (36.61 g, 162 mmol, 1.2 eq). The mixture was reacted at 93°C for 2 hours. After the reaction was completed, the mixture was filtered, and the filtrate was concentrated. It was first purified by normal-phase column chromatography (dichloromethane:methanol = 95:5), then further purified by reverse-phase column chromatography. The target product was eluted when the ratio of acetonitrile to water (containing 1‰ formic acid) was 13:87, and then concentrated to obtain (S)-1-{[6-bromo-2-(difluoromethyl)pyridin-3-yl]oxy}-2,4-dimethylpentan-2-amine as a pale yellow oily product (11.80 g, yield 25.88%). LCMS(TOF MS ES+)m/z [M+H]⁺: 337/339. ¹H NMR (400 MHz, DMSO) δ 7.79 (d, J = 8.8 Hz, 1H), 7.66 (d, J = 8.9 Hz, 1H), 7.16 (t, J = 53.4 Hz, 1H), 3.81 (s, 2H), 1.79 (m, J = 12.7, 6.3 Hz, 1H), 1.38 - 1.34 (m, 2H), 1.10 (s, 3H), 0.92 (m, J = 8.4, 6.6 Hz, 6H).

Step 4: The compound (S)-1-{[6-bromo-2-(difluoromethyl)pyridin-3-yl]oxy}-2,4-dimethylpentan-2-amine (450 mg, 1.35 mmol, 1.0 eq) and bis(pinacolato)diboron (405 mg, 1.59 mmol, 1.2 eq) were dissolved in 1,4-dioxane (15.0 mL), followed by the addition of potassium acetate (393 mg, 4.02 mmol, 3.0 eq) and [1,1'-bis(diphenylphosphino)ferrocene]palladium dichloride (58.5 mg, 0.08 mmol, 0.06 eq). The mixture was reacted at 80°C for 16 hours under a nitrogen atmosphere. After the reaction was completed, the mixture was filtered to obtain (S)-{5-[(2-amino-2,4-dimethylpentyl)oxy]-6-(difluoromethyl)pyridin-2-yl}boronic acid (4.8 mL) as a crude product filtrate. Then, 5-fluoro-3-iodo-1-p-tosyl-1H-pyrrolo[2,3-b]pyridine (588 mg, 1.35 mmol, 1.0 eq), [1,1'-bis(diphenylphosphino)ferrocene]palladium dichloride (99.0 mg, 0.135 mmol, 0.1 eq), and sodium carbonate (426 mg, 4.02 mmol, 3.0 eq) were added to the above solution. The mixture was reacted at 90°C for 4 hours under a nitrogen atmosphere. After the reaction was completed, the mixture was filtered through diatomaceous earth. The filtrate was concentrated and purified by normal-phase column chromatography (dichloromethane:methanol = 15:1) to obtain (S)-1-{[2-(difluoromethyl)-6-(5-fluoro-1-tosyl-1H-pyrrolo[2,3-b]pyridin-3-yl)pyridin-3-yl]ox y}-2,4-dimethylpentan-2-amine as a brown solid product (260.7 mg, yield 35.75%). ¹H NMR (400 MHz, DMSO) δ 8.81 (s, 1H), 8.71 (dd, J = 9.3, 2.9 Hz, 1H), 8.48 (dd, J = 2.9, 1.2 Hz, 1H), 8.34 (d, J = 8.8 Hz, 1H), 8.04 (d, J = 8.4 Hz, 2H), 7.78 (d, J = 8.9 Hz, 1H), 7.50 - 7.17 (m, 3H), 3.95 (s, 2H), 2.37 (s, 3H), 1.82 (dt, J = 12.7, 6.3 Hz, 1H), 1.43 - 1.39 (m, 2H), 1.33 (d, J = 9.8 Hz, 1H), 1.22 - 1.17 (m, 1H), 1.14 (s, 3H), 0.94 (dd, J = 8.7, 6.6 Hz, 6H).

Step 5: (S)-1-{[2-(Difluoromethyl)-6-(5-fluoro-1-tosyl-1H-pyrrolo[2,3-b]pyridin-3-yl)pyridin-3-yl]o xy}-2,4-dimethylpentan-2-amine (476 mg, 1.21 mmol, 1.0 eq) was added to a solution of hydrogen chloride in dioxane (3 mL, 4.0 mol/L), and the mixture was reacted at 60°C for 16 hours. After the reaction was completed, the mixture was directly filtered. The filter cake was washed with a mixed solvent (petroleum ether:ethyl acetate = 5:1, 20 mL) to remove excess impurities. The filter cake was collected and dried under reduced pressure to obtain (S)-1-{[2-(difluoromethyl)-6-(5-fluoro-1H-pyrrolo[2,3-b]pyridin-3-yl)pyridin-3-yl]oxy}-2,4-dimethylpentan-2-amine trihydrochloride (246 mg, yield 40.46%). LCMS(TOF MS ES+)m/z [M+H]⁺: 393.19. ¹H NMR (400 MHz, DMSO) δ 12.22 (d, *J =* 2.9 Hz, 1H), 8.59 (dd, *J =* 9.9, 2.9 Hz, 1H), 8.39 (d, *J =* 2.9 Hz, 4H), 8.29 (dd, *J =* 2.9, 1.5 Hz, 1H), 8.09 (d, *J=* 8.9 Hz, 1H), 7.81 - 7.46 (m, 2H), 4.25 - 4.13 (m, 2H), 1.86 - 1.72 (m, 2H), 1.62 (dd, *J =* 14.0, 5.3 Hz, 1H), 1.39 (s, 3H), 0.96 (d, *J =* 6.5 Hz, 3H), 0.91 (d, *J =* 6.4 Hz, 3H).

Step 6: Dissolve (S)-1-{[2-(difluoromethyl)-6-(5-fluoro-1H-pyrrolo[2,3-b]pyridin-3-yl)pyridin-3-yl]oxy}-2,4-dimethylpentan-2-amine trihydrochloride (100 mg, 0.2 mmol, 1.0 eq) in tetrahydrofuran (1 mL), then add this solution to an aqueous solution (1 mL) of sodium hydroxide (24 mg, 0.6 mmol, 3 eq), and react at room temperature for 1 hour. After the reaction is completed, directly perform preparative separation to obtain a white solid (S)-1-{[2-(difluoromethyl)-6-(5-fluoro-1H-pyrrolo[2,3-b]pyridin-3-yl)pyridin-3-yl]oxy}-2,4-dimethylpentan-2-amine (31.7 mg, yield 40.56%). LCMS (TOF MS ES+) m/z [M+H]+: 393.19.

### Example 28: Synthesis of (S)-1-{[2-(difluoromethyl)-6-[5-(difluoromethyl)-1H-pyrrolo[2,3-b]pyridin-3-yl]pyridin-3-yl]oxy}-2,4-dimethylpentan-2-amine formate (Compound 28)

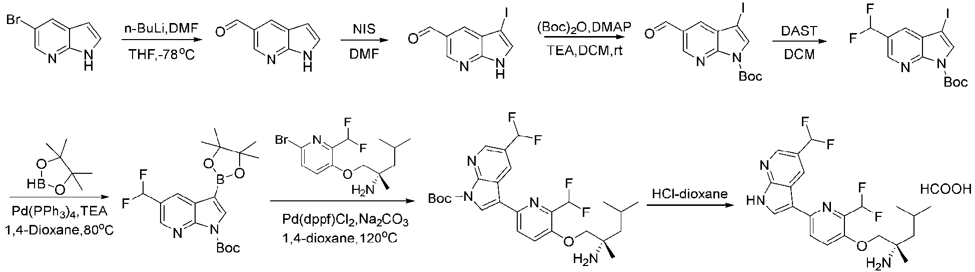

### Step 1: Synthesis of 7-azaindole-5-carbaldehyde

5-Bromo-7-azaindole (5 g, 25 mmol, 1.0 eq) was dissolved in tetrahydrofuran (50 mL). Under a nitrogen atmosphere, n-butyllithium (21 mL, 2.5 mol/L, 2.1 eq) was slowly added dropwise at -78°C. The mixture was reacted at -78°C for 40 minutes, and then anhydrous N,N-dimethylformamide (5 mL) was slowly added dropwise at this temperature. The mixture was reacted at room temperature for 2 hours. After the reaction was completed, water (200 mL) and ethyl acetate (200 mL) were added for extraction and liquid separation. The organic phase was washed three times with water (150 mL), dried over anhydrous sodium sulfate, and purified by normal-phase column chromatography (petroleum ether:ethyl acetate = 2:3) to obtain 7-azaindole-5-carbaldehyde as a yellow solid (2.4 g, yield 64.43%). LCMS(TOF MS ES+)m/z [M+H]+: 147. ¹H NMR (400 MHz, DMSO) δ 12.20 (s, 1H), 10.11 (s, 1H), 8.78 (d, *J =* 2.0 Hz, 1H), 8.50 (d, *J =* 1.9 Hz, 1H), 7.67 (d, *J =* 3.5 Hz, 1H), 6.69 (d, *J* = 3.5 Hz, 1H).

### Step 2: Synthesis of 3-iodo-1H-pyrrolo[2,3-b]pyridine-5-carbaldehyde

The compound 7-azaindole-5-carbaldehyde (2.4 g, 16.42 mmol, 1.0 eq) was dissolved in N,N-dimethylformamide (25 mL). N-Iodosuccinimide (3.69 g, 16.42 mmol, 1.0 eq) was slowly added at 0°C, and the mixture was reacted at room temperature for 16 hours. After the reaction was completed, the reaction solution was diluted with ethyl acetate (200 mL), and washed three times with water (200 mL) and once with saturated aqueous sodium chloride. The organic phase was dried over anhydrous sodium sulfate, filtered, concentrated, and purified by normal-phase column chromatography (petroleum ether:ethyl acetate = 3:1) to obtain 3-iodo-1H-pyrrolo[2,3-b]pyridine-5-carbaldehyde as a yellow solid (4.0 g, yield 89.55%). LCMS(TOF MS ES+)m/z [M+H]+: 272.94. 1H NMR (400 MHz, DMSO) δ 12.65 (s, 1H), 10.17 (s, 1H), 8.81 (d, J = 1.9 Hz, 1H), 8.22 (d, J = 1.9 Hz, 1H), 7.94 (d, J = 2.5 Hz, 1H).

### Step 3: Synthesis of tert-butyl 5-formyl-3-iodo-1H-pyrrolo[2,3-b]pyridine-1-carboxylate

3-Iodo-1H-pyrrolo[2,3-b]pyridine-5-carbaldehyde (2 g, 7.35 mmol, 1.0 eq) and triethylamine (3.06 mL, 22.06 mmol, 3.0 eq) were dissolved in dichloromethane (25 mL). 4-Dimethylaminopyridine (45 mg, 0.37 mmol, 0.05 eq) and di-tert-butyl dicarbonate (1.76 g, 8.09 mmol, 1.1 eq) were added at 0°C, and the mixture was reacted at room temperature for 0.5 hours. After the reaction was completed, extraction and liquid separation were performed with ethyl acetate and water. The organic layer was collected, and washed twice with water and once with saturated brine. Then, the organic phase was dried over anhydrous sodium sulfate, filtered, and purified by normal-phase column separation. When the ethyl acetate content was 20%, tert-butyl 5-formyl-3-iodo-1H-pyrrolo[2,3-b]pyridine-1-carboxylate was obtained as a white solid (1.7 g, yield 62.16%). LCMS(TOF MS ES+)m/z [M+H]+: 373. ¹H NMR (400 MHz, DMSO) δ 10.23 (s, 1H), 8.96 (d, *J= 1.9* Hz, 1H), 8.26 (d, *J=* 1.9 Hz, 1H), 8.20 (s, 1H), 1.64 (s, 9H).

### Step 4: Synthesis of tert-butyl 5-(difluoromethyl)-3-iodo-1H-pyrrolo [2,3-b] pyridine-1-carboxylate

Tert-butyl 5-formyl-3-iodo-1H-pyrrolo[2,3-b]pyridine-1-carboxylate (1.0 g, 2.69 mmol, 1.0 eq) was dissolved in dichloromethane (15 mL). Diethylaminosulfur trifluoride (866 mg, 5.38 mmol, 2.0 eq) was added dropwise slowly at 0°C. Subsequently, the mixture was allowed to react at room temperature for 5 hours. After the reaction was completed, the mixture was extracted with ethyl acetate and water. The organic phase was washed three times with water and once with saturated sodium chloride solution. The organic phase was then dried over anhydrous sodium sulfate, filtered, concentrated, and purified by normal-phase silica gel column chromatography (petroleum ether:ethyl acetate = 4:1) to obtain tert-butyl 5-(difluoromethyl)-3-iodo-1H-pyrrolo[2,3-b]pyridine-1-carboxylate as a pale yellow solid (750 mg, yield 70.69%). LCMS(TOF MS ES+)m/z [M+H]+: 395. ¹H NMR (400 MHz, DMSO) δ 8.69 - 8.64 (m, 1H), 8.17 (s, 1H), 8.00 (dt, *J = 2.2,* 1.2 Hz, 1H), 7.47 - 7.14 (m, 1H), 1.63 (s, 9H).

### Step 5: Synthesis of tert-butyl 5-(difluoromethyl)-3-(4,4,5,5-tetramethyl-1,3,2-dioxaborolan-2-yl)-1H-pyrrolo[2,3-b]pyr idine-1-carboxylate

The compounds tert-butyl 5-(difluoromethyl)-3-iodo-1H-pyrrolo[2,3-b]pyridine-1-carboxylate (750 mg, 1.90 mmol, 1.0 eq), 4,4,5,5-tetramethyl-1,3,2-dioxaborolane (414 mg, 3.23 mmol, 1.7 eq), and triethylamine (963 mg, 9.51 mmol, 5.0 eq) were added to 1,4-dioxane (10.0 mL). Finally, tetrakis(triphenylphosphine)palladium (220 mg, 0.19 mmol, 0.1 eq) was added, and the mixture was reacted at 80°C for 16 hours under a nitrogen atmosphere. After the reaction was completed, the mixture was filtered, and the filtrate was concentrated and purified by normal-phase column chromatography to obtain tert-butyl 5-(difluoromethyl)-3-(4,4,5,5-tetramethyl-1,3,2-dioxaborolan-2-yl)-1H-pyrrolo[2,3-b]pyridin e-1-carboxylate as a yellow solid product (115 mg, yield 15.33%). LCMS(TOF MS ES+)m/z [M+H]+: 395. ¹H NMR (400 MHz, DMSO) δ 8.63 (m, *J =* 1.5 Hz, 1H), 8.33 - 8.26 (m, 1H), 8.10 (s, 1H), 7.46 - 7.11 (m, 1H), 1.62 (s, 9H), 1.33 (s, 12H).

### Step 6: Synthesis of tert-butyl (S)-3-{5-[(2-amino-2,4-dimethylpentyl)oxy]-6-(difluoromethyl)pyridin-2-yl}-5-(difluoro methyl)-1H-pyrrolo[2,3-b]pyridine-1-carboxylate

(S)-1-{[6-bromo-2-(difluoromethyl)pyridin-3-yl]oxy}-2,4-dimethylpentan-2-a mine (85 mg, 0.25 mmol, 1.0 eq), tert-butyl 5-(difluoromethyl)-3-(4,4,5,5-tetramethyl-1,3,2-dioxaborolan-2-yl)-1H-pyrrolo[2,3-b]pyridin e-1-carboxylate (100 mg, 0.25 mmol, 1.0 eq), sodium carbonate (107 mg, 1.00 mmol, 4.0 eq), and [1,1'-bis(diphenylphosphino)ferrocene]palladium dichloride (18 mg, 0.025 mmol, 0.10 eq) were added to 1,4-dioxane (5.0 mL). The mixture was reacted at 120°C for 8 hours under a nitrogen atmosphere. After the reaction was completed, the mixture was filtered, and the filtrate was concentrated and purified by reverse-phase C18 column chromatography to obtain tert-butyl (S)-3-{5-[(2-amino-2,4-dimethylpentyl)oxy]-6-(difluoromethyl)pyridin-2-yl}-5-(difluoromet hyl)-1H-pyrrolo[2,3-b]pyridine-1-carboxylate as a yellow solid product (45 mg, yield 33.83%). LCMS(TOF MS ES+)m/z [M+H]+: 525.24. ¹H NMR (400 MHz, DMSO) δ 9.21 (d, *J* = 2.2 Hz, 1H), 8.69 (s, 1H), 8.66 (s, 1H), 8.32 (d, *J =* 8.8 Hz, 1H), 7.77 (d, *J =* 8.8 Hz, 1H), 7.49 - 7.16 (m, 2H), 3.91 (s, 2H), 1.82 (dd, *J =* 12.7, 6.3 Hz, 1H), 1.69 (s, 9H), 1.50 - 1.38 (m, 2H), 1.16 (s, 3H), 0.95 (dd, *J = 9.1,* 6.6 Hz, 6H).

### Step 7: Synthesis of (S)-1-{[2-(difluoromethyl)-6-[5-(difluoromethyl)-1H-pyrrolo[2,3-b]pyridin-3-yl]pyridin-3-yl]oxy}-2,4-dimethylpentan-2-amine formate

Tert-butyl (S)-3-{5-[(2-amino-2,4-dimethylpentyl)oxy]-6-(difluoromethyl)pyridin-2-yl}-5-(difluoromet hyl)-1H-pyrrolo[2,3-b]pyridine-1-carboxylate (45 mg, 0.086 mmol, 1.0 eq) was added to a solution of hydrogen chloride in dioxane (3.0 mL, 4.0 mol/L). The mixture was reacted at room temperature for 2 hours. After the reaction was completed, the mixture was directly concentrated and purified by reverse-phase preparative chromatography on a C18 column (formic acid system) to obtain (S)-1-{[2-(difluoromethyl)-6-[5-(difluoromethyl)-1H-pyrrolo[2,3-b]pyridin-3-yl]pyridin-3-yl ]oxy}-2,4-dimethylpentan-2-amine formate as a final product (32.3 mg, yield 80.03%). LCMS(TOF MS ES+)m/z [M+H]+: 425.2298. ¹H NMR (400 MHz, DMSO) δ 12.34 (s, 1H), 9.08 (s, 1H), 8.50 (s, 1H), 8.40 (s, 1H), 8.30 (s, 1H), 8.11 (d, *J =* 8.8 Hz, 1H), 7.73 (d, *J =* 8.9 Hz, 1H), 7.52 - 7.10 (m, 2H), 3.97 (s, 2H), 1.83 (m, *J =* 12.7, 6.4 Hz, 1H), 1.51 (m, *J =* 14.1, 5.6 Hz, 2H), 1.23 (s, 3H), 0.95 (dd, *J =* 12.6, 6.6 Hz, 6H).

### Example 43: Synthesis of (S)-1-{[2-(difluoromethyl)-6-(5-fluoro-1-methyl-1H-pyrrolo[2,3-b]pyridin-3-yl)pyridin-3 -yl]oxy}-2,4-dimethylpentan-2-amine (Compound 43)

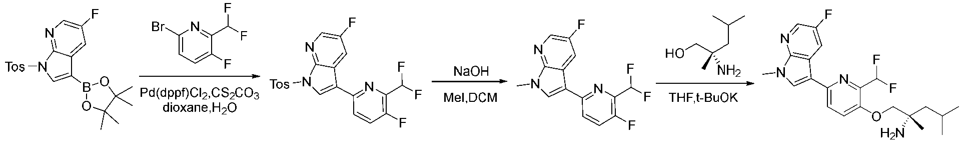

### Step 1: Synthesis of 3-[6-(difluoromethyl)-5-fluoropyridin-2-yl]-5-fluoro-1-tosyl-1H-pyrrolo[2,3-b]pyridine

5-Fluoro-3-(4,4,5,5-tetramethyl-1,3,2-dioxaborolan-2-yl)-1-tosyl-1H-pyrrolo[2 ,3-b]pyridine (200 mg, 0.48 mmol, 1.0 eq), 6-bromo-2-(difluoromethyl)-3-fluoropyridine (108 mg, 0.48 mmol, 1.0 eq), and [1,1'-bis(diphenylphosphino)ferrocene]palladium dichloride (35 mg, 0.048 mmol, 0.1 eq) were added to a mixed solvent of 1,4-dioxane (4 mL) and water (1 mL). The mixture was reacted at 90°C for 4 hours. After the reaction was completed, the mixture was cooled and extracted 3 times with ethyl acetate (10 mL). The organic phase was concentrated and purified by normal-phase chromatography (PE:EA = 3:1) to obtain 3-[6-(difluoromethyl)-5-fluoropyridin-2-yl]-5-fluoro-1-tosyl-1H-pyrrolo[2,3-b]pyridine as a yellow solid (135 mg, yield 64.6%). LCMS (TOF MS ES+) m/z [M+H]+: 436.1. ¹H NMR (400 MHz, DMSO) δ 8.95 (s, 1H), 8.72 (dd, J = 9.2, 2.8 Hz, 1H), 8.50 (m, J = 5.9, 4.1 Hz, 2H), 8.05 (d, J = 8.3 Hz, 2H), 7.46 (d, J = 8.2 Hz, 2H), 7.38 - 7.21 (m, 1H), 2.37 (s, 3H).

### Step 2: Synthesis of 3-[6-(difluoromethyl)-5-fluoropyridin-2-yl]-5-fluoro-1-methyl-1H-pyrrolo[2,3-b]pyridin e

3-[6-(Difluoromethyl)-5-fluoropyridin-2-yl]-5-fluoro-1-tosyl-1H-pyrrolo[2,3-b ]pyridine (135 mg, 0.31 mmol, 1.0 eq), sodium hydroxide (49.6 mg, 1.24 mmol, 4.0 eq), and methyl iodide (49.6 mg, 0.93 mmol, 3.0 eq) were added to dichloromethane (5 mL). The mixture was then heated to 40°C and reacted for 16 hours. After the reaction was completed, the mother liquor was concentrated and purified by normal-phase chromatography (PE:EA = 4:1) to obtain 3-[6-(difluoromethyl)-5-fluoropyridin-2-yl]-5-fluoro-1-methyl-1H-pyrrolo[2,3-b]pyridine as a yellow solid (65.0 mg, yield 71.0%). LCMS(TOF MS ES+)m/z [M+H]+: 296. 1H NMR (400 MHz, DMSO) δ 8.64 (dd, J = 9.7, 2.8 Hz, 1H), 8.53 (s, 1H), 8.44-8.34 (m, 1H), 8.12 (dd, J = 8.9, 3.7 Hz, 1H), 7.97 (t, J = 9.5 Hz, 1H), 7.49 - 7.16 (m, 1H), 3.92 (s, 3H).

### Step 3: Synthesis of (S)-1-{[2-(difluoromethyl)-6-(5-fluoro-1-methyl-1H-pyrrolo[2,3-b]pyridin-3-yl)pyridin-3 -yl]oxy}-2,4-dimethylpentan-2-amine

3-[6-(Difluoromethyl)-5-fluoropyridin-2-yl]-5-fluoro-1-methyl-1H-pyrrolo[2,3 -b]pyridine (88 mg, 0.22 mmol, 1.0 eq) and (S)-2-amino-2,4-dimethylpentan-1-ol (29 mg, 0.22 mmol, 1.0 eq) were added to tetrahydrofuran (2 mL), followed by the addition of potassium tert-butoxide (74 mg, 0.66 mmol, 3.0 eq). The mixture was reacted at 90°C for 4 hours. After the reaction was completed, the mixture was filtered, and the mother liquor was concentrated and purified by preparative chromatography to obtain (S)-1-{[2-(difluoromethyl)-6-(5-fluoro-1-methyl-1H-pyrrolo[2,3-b]pyridin-3-yl)pyridin-3-yl] oxy}-2,4-dimethylpentan-2-amine as a white solid (6.9 mg, yield 7.8%). LCMS(TOF MS ES+)m/z [M+H]+: 407.20. 1H NMR (400 MHz, DMSO) 1H NMR (400 MHz, DMSO) δ 8.60 (dd, J = 9.8, 2.8 Hz, 1H), 8.38 (s, 1H), 8.33 (dd, J = 2.9, 1.5 Hz, 1H), 7.96 (d, J = 8.8 Hz, 1H), 7.67 (d, J = 8.9 Hz, 1H), 7.40-7.09 (m, 1H), 3.88 (s, 3H), 3.81 (s, 2H), 1.80 (m, J = 12.6, 6.2 Hz, 1H), 1.38 (dd, J = 5.5, 2.4 Hz, 2H), 1.11 (s, 3H), 0.92 (t, J = 7.0 Hz, 6H).

### Example 44: Synthesis of (S)-1-{[6-(5-chloro-1H-pyrrolo[2,3-b]pyridin-3-yl)-2-(difluoromethyl)pyridin-3-yl]oxy}-2 ,4-dimethylpentan-2-amine (Compound 44)

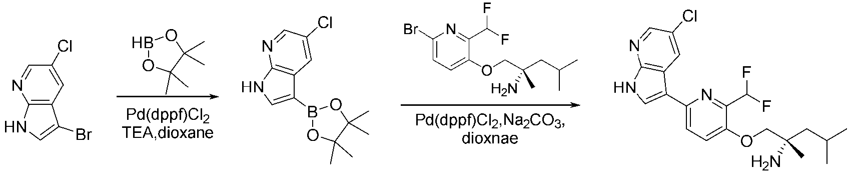

### Step 1: Synthesis of 5-chloro-3-(4,4,5,5-tetramethyl-1,3,2-dioxaborolan-2-yl)-1H-pyrrolo[2,3-b]pyridine

Compounds 3-bromo-5-chloro-1H-pyrrolo[2,3-b]pyridine (1.0 g, 4.35 mmol, 1.0 eq), 4,4,5,5-tetramethyl-1,3,2-dioxaborolane (2.2 g, 17.2 mmol, 4.0 eq), [1,1'-bis(diphenylphosphino)ferrocene]palladium dichloride (159 mg, 0.217 mmol, 0.05 eq), and triethylamine (2.19 g, 2.17 mmol, 5 eq) were added to 1,4-dioxane (10 mL). The mixture was reacted at 120°C for 1 hour under a nitrogen atmosphere. After the reaction was completed, insoluble materials were removed by filtration, and the filtrate was separated and purified by reverse-phase column chromatography to obtain 5-chloro-3-(4,4,5,5-tetramethyl-1,3,2-dioxaborolan-2-yl)-1H-pyrrolo[2,3-b]pyridine as an off-white solid (155 mg, yield 12.7%). LCMS(ESI)[M+H]+: 279. ¹H NMR (400 MHz, CDCl3) δ 8.37 (dd, J = 4.8, 1.6 Hz, 1H), 8.31 (s, J = 7.8, 1.6 Hz, 1H), 7.68 (s, 1H) , 1.39 (s, 12H).

### Step 2: Synthesis of (S)-1-{[6-(5-chloro-1H-pyrrolo[2,3-b]pyridin-3-yl)-2-(difluoromethyl)pyridin-3-yl]oxy}-2 ,4-dimethylpentan-2-amine

5-Chloro-3-(4,4,5,5-tetramethyl-1,3,2-dioxaborolan-2-yl)-1H-pyrrolo[2,3-b]py ridine (50 mg, 0.180 mmol, 1.0 eq) and (S)-1-{[6-bromo-2-(difluoromethyl)pyridin-3-yl]oxy}-2,4-dimethylpentan-2-amine (60.4 mg, 0.180 mmol, 1.0 eq) were dissolved in 1,4-dioxane (2 mL), followed by the addition of [1,1'-bis(diphenylphosphino)ferrocene]palladium dichloride (13.2 mg, 0.018 mmol, 0.05 eq) and sodium carbonate (57.2 mg, 0.54 mmol, 3.0 eq). The mixture was reacted at 120°C for 2 hours under a nitrogen atmosphere. After the reaction was completed, excess solids were filtered off, and the filtrate was concentrated and purified by preparative chromatography to obtain (S)-1-{[6-(5-chloro-1H-pyrrolo[2,3-b]pyridin-3-yl)-2-(difluoromethyl)pyridin-3-yl]oxy}-2,4-dimethylpentan-2-amine as an off-white solid (4.3 mg, yield 5.8%). LCMS (ESI) [M+H]+: 409.19. 1H NMR (400 MHz, DMSO-d6) δ 12.32 (d, J = 2.9 Hz, 1H), 8.89 (d, J = 2.4 Hz, 1H), 8.42 (d, J = 2.8 Hz, 1H), 8.32 (d, J = 2.4 Hz, 1H), 8.14 (s, 1H), 8.11 (s, 2H), 7.79 (d, J = 9.0 Hz, 1H), 7.71 - 7.41 (m, 1H), 4.25 - 4.13 (m, 2H), 1.89 - 1.72 (m, 2H), 1.61 (dd, J = 14.1, 5.3 Hz, 1H), 1.40 (s, 3H), 1.00 (d, J = 6.5 Hz, 3H), 0.94 (d, J = 6.5 Hz, 3H).

### Example 45: Synthesis of (S)-1-{[2-(difluoromethyl)-6-(5-methyl-1H-pyrrolo[2,3-b]pyridin-3-yl)pyridin-3-yl]oxy}-2,4-dimethylpentan-2-amine diformate (Compound 45)

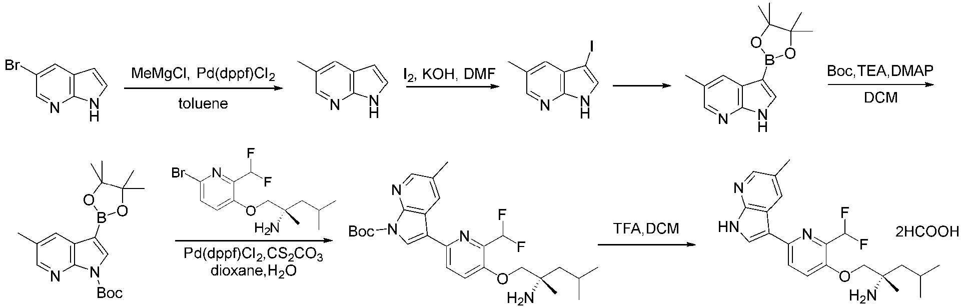

### Step 1: Synthesis of 5-methyl-1H-pyrrolo[2,3-b]pyridine

Compounds 5-Bromo-1H-pyrrolo[2,3-b]pyridine (11.0 g, 33.7 mmol, 1.0 eq) and [1,1'-bis(diphenylphosphino)ferrocene]palladium dichloride (197.3 mg, 0.27 mmol, 0.008 eq) were added to toluene (100 mL). Under a nitrogen atmosphere, the mixture was heated to 100°C, followed by the dropwise addition of methylmagnesium chloride (3 M, 28.2 mL, 2.5 eq) to the reaction system. The reaction was continued for 2 hours. After the reaction was completed, the reaction solution was directly concentrated. Water and ethyl acetate were added for extraction and liquid separation. The organic phase was concentrated and purified by normal-phase column separation (PE:EA = 4:1) to obtain 5-methyl-1H-pyrrolo[2,3-b]pyridine as a colorless oily product (4.0 g, yield 53.95%). LCMS(ESI)[M+H]+: 133.

### Step 2: Synthesis of 3-iodo-5-methyl-1H-pyrrolo[2,3-b]pyridine

5-Methyl-1H-pyrrolo[2,3-b]pyridine (4.0 g, 30.26 mmol, 1.0 eq) and iodine (3.84 g, 15.13 mmol, 0.5 eq) were dissolved in N,N-dimethylformamide (30 mL). Potassium hydroxide (8.56 g, 151.3 mmol, 5 eq) was added in portions, and the mixture was reacted at room temperature for 16 hours. After the reaction was completed, the reaction solution was poured into ice water, and a yellow solid was formed. The solid was filtered, and the filter cake was collected and dried to obtain 3-iodo-5-methyl-1H-pyrrolo[2,3-b]pyridine as a yellow crude product (3.0 g). LCMS(ESI)[M+H]+: 259.

### Step 3: Synthesis of 5-methyl-3-(4,4,5,5-tetramethyl-1,3,2-dioxaborolan-2-yl)-1H-pyrrolo[2,3-b]pyridine

3-Iodo-5-methyl-1H-pyrrolo[2,3-b]pyridine (3.0 g, 11.64 mmol, 1.0 eq), 4,4,5,5-tetramethyl-1,3,2-dioxaborolane (7.5 g, 58.2 mmol, 5.0 eq), triethylamine (5.96 g, 58.2 mmol, 5.0 eq), and [1,1'-bis(diphenylphosphino)ferrocene]palladium dichloride (852 mg, 1.164 mmol, 0.1 eq) were added to 1,4-dioxane (15 mL). The mixture was reacted at 120°C for 2 hours under a nitrogen atmosphere. The mixture was filtered through diatomaceous earth, and the filtrate was concentrated and purified by normal-phase column separation to obtain 5-methyl-3-(4,4,5,5-tetramethyl-1,3,2-dioxaborolan-2-yl)-1H-pyrrolo[2,3-b]pyridine as a yellow solid (2.0 g). LCMS(ESI)[M+H]+: 259. ¹H NMR (400 MHz, CDCl3) δ 8.05 (dd, J = 7.9, 2.0 Hz, 1H), 7.93 (s, 2H), 7.72 (d, J = 2.3 Hz, 1H), 1.30 (s, 3H), 1.07 (s, 12H).

### Step 4: Synthesis of tert-butyl 5-methyl-3-(4,4,5,5-tetramethyl-1,3,2-dioxaborolan-2-yl)-1H-pyrrolo[2,3-b]pyridine-1-c arboxylate

5-Methyl-3-(4,4,5,5-tetramethyl-1,3,2-dioxaborolan-2-yl)-1H-pyrrolo[2,3-b]p yridine (100 mg, 0.384 mmol, 1 eq) was dissolved in dichloromethane (5 mL). Di-tert-butyl dicarbonate (110 mg, 0.504 mmol, 1.3 eq), triethylamine (78.4 mg, 0.775 mmol, 2.0 eq), and DMAP (4.7 mg, 0.038 mmol, 0.1 eq) were sequentially added at 0°C. The reaction was maintained at 0°C for 1 hour. After the reaction was completed, water (10 mL) was added for dilution, and the mixture was extracted three times with 20 mL of dichloromethane. The organic phase was dried over anhydrous sodium sulfate, concentrated, and purified by a TLC preparative plate (PE:EA = 2:1) to obtain tert-butyl 5-methyl-3-(4,4,5,5-tetramethyl-1,3,2-dioxaborolan-2-yl)-1H-pyrrolo[2,3-b]pyridine-1-carbo xylate as a yellow oil (81 mg, yield: 58.4%). LCMS(ESI)[M+H]+: 359.

### Step 5: Synthesis of tert-butyl (S)-3-{5-[(2-amino-2,4-dimethylpentyl)oxy]-6-(difluoromethyl)pyridin-2-yl}-5-methyl-1 H-pyrrolo[2,3-b]pyridine-1-carboxylate

Tert-butyl 5-methyl-3-(4,4,5,5-tetramethyl-1,3,2-dioxaborolan-2-yl)-1H-pyrrolo[2,3-b]pyridine-1-carbo xylate (81 mg, 0.226 mmol, 1.0 eq) and (S)-1-{[6-bromo-2-(difluoromethyl)pyridin-3-yl]oxy}-2,4-dimethylpentan-2-amine (75.8 mg, 0.226 mmol, 1.0 eq) were dissolved in 1,4-dioxane (2 mL) and water (0.5 mL), followed by the addition of [1,1'-bis(diphenylphosphino)ferrocene]palladium dichloride (16.8 mg, 0.023 mmol, 0.1 eq) and cesium carbonate (222.3 mg, 0.678 mmol, 3.0 eq). The mixture was reacted at 80°C for 2 hours under a nitrogen atmosphere. After the reaction was completed, the mixture was filtered through diatomaceous earth, and the filtrate was concentrated and purified by reverse-phase column chromatography (50% acetonitrile) to obtain tert-butyl (S)-3-{5-[(2-amino-2,4-dimethylpentyl)oxy]-6-(difluoromethyl)pyridin-2-yl}-5-methyl-1H-p yrrolo[2,3-b]pyridine-1-carboxylate as an off-white solid (33 mg, crude). The product was used directly in the next step without further purification. LCMS(ESI)[M+H]+: 489.

### Step 6: Synthesis of (S)-1-{[2-(difluoromethyl)-6-(5-methyl-1H-pyrrolo[2,3-b]pyridin-3-yl)pyridin-3-yl]oxy}-2,4-dimethylpentan-2-amine diformate

The compound tert-butyl (S)-3-{5-[(2-amino-2,4-dimethylpentyl)oxy]-6-(difluoromethyl)pyridin-2-yl}-5-methyl-1H-p yrrolo[2,3-b]pyridine-1-carboxylate (33 mg, crude) was added to dichloromethane (2 mL), followed by the addition of trifluoroacetic acid (0.5 mL). The mixture was reacted at room temperature for 1 hour. After the reaction was completed, the organic phase was concentrated and purified by preparative chromatography to obtain (S)-1-{[2-(difluoromethyl)-6-(5-methyl-1H-pyrrolo[2,3-b]pyridin-3-yl)pyridin-3-yl]oxy}-2,4 -dimethylpentan-2-amine diformate as a white solid (9.3 mg, yield 35.4%). LCMS (ESI) [M+H]+: 389.21. ¹H NMR (400 MHz, DMSO) δ 11.92 (d, J = 2.9 Hz, 1H), 8.65 (d, J = 2.1 Hz, 1H), 8.21 (d, J = 2.8 Hz, 1H), 8.14 (d, J = 2.1 Hz, 1H), 8.13 (s, 2H), 8.06 (d, J = 8.9 Hz, 1H), 7.73 (d, J = 8.9 Hz, 1H), 7.66-7.36 (m, 1H), 4.73 (s, 2H), 4.21-4.10 (m, 2H), 2.42 (s, 3H), 1.87-1.69 (m, 2H), 1.60 (dd, J = 14.1, 5.3 Hz, 1H), 1.38 (s, 3H), 1.00-0.89 (m, 6H).

### Example 46: Synthesis of (S)-1-{[3-(difluoromethyl)-5-(1H-pyrrolo[2,3-b]pyridin-3-yl)pyridin-2-yl]oxy}-2,4-dimet hylpentan-2-amine formate (Compound 46)

### Step 1: Synthesis of (S)-1-{[5-bromo-3-(difluoromethyl)pyridin-2-yl]oxy}-2,4-dimethylpentan-2-amine

A compound (S)-2-amino-2,4-dimethylpentan-1-ol (275 mg, 2.10 mmol, 0.95 eq) was added to tetrahydrofuran (6.0 mL), followed by the addition of potassium tert-butoxide (620 mg, 5.53 mmol, 2.5 eq) and 5-bromo-3-(difluoromethyl)-2-fluoropyridine (500 mg, 2.21 mmol, 1.0 eq). The mixture was reacted at 93°C for 2 hours. After the reaction was completed, the mixture was filtered, and the filtrate was concentrated and purified by normal-phase column chromatography (petroleum ether:ethyl acetate = 3:1) to obtain (S)-1-{[5-bromo-3-(difluoromethyl)pyridin-2-yl]oxy}-2,4-dimethylpentan-2-amine as a yellow oily product (200 mg, yield 26.85%). LCMS(TOF MS ES+)m/z [M+H]+: 337/339. 1H NMR (400 MHz, DMSO) δ 8.49 - 8.43 (m, 1H), 8.14 (d, *J=* 2.4 Hz, 1H), 7.31 - 7.00 (m, 1H), 4.04 (d, *J =* 1.7 Hz, 2H), 1.79 (m, *J =* 12.8, 6.4 Hz, 1H), 1.60 (s, 2H), 1.35 (m, *J =* 5.8, 3.4 Hz, 2H), 1.09 (s, 3H), 0.90 (d, *J =* 6.7 Hz, 6H).

### Step 2: Synthesis of (S)-1-{[3-(difluoromethyl)-5-(1H-pyrrolo[2,3-b]pyridin-3-yl)pyridin-2-yl]oxy}-2,4-dimet hylpentan-2-amine formate

(S)-1-{[5-bromo-3-(difluoromethyl)pyridin-2-yl]oxy}-2,4-dimethylpentan-2-a mine (80 mg, 0.24 mmol, 1.0 eq), 7-azaindole-3-boronic acid pinacol ester (57.91 mg, 0.24 mmol, 1.0 eq), sodium carbonate (100 mg, 0.95 mmol, 4.0 eq), and [1,1'-bis(diphenylphosphino)ferrocene]palladium dichloride (17.56 mg, 0.024 mmol, 0.10 eq) were added to 1,4-dioxane (3.0 mL). The mixture was reacted at 120°C for 8 hours under a nitrogen atmosphere. After the reaction was completed, the mixture was filtered, and the filtrate was purified by reverse-phase chromatography on a C18 column (formic acid system) to obtain (S)-1-{[3-(difluoromethyl)-5-(1H-pyrrolo[2,3-b]pyridin-3-yl)pyridin-2-yl]oxy}-2,4-dimethyl pentan-2-amine formate as a white solid (6.0 mg, yield 6.02%). LCMS(TOF MS ES+)m/z [M+H]+: 375/376. 1H NMR (400 MHz, DMSO) δ 12.07 (s, 1H), 8.70 (d, J = 2.3 Hz, 1H), 8.44-8.36 (m, 1H), 8.31 (dd, J = 4.7, 1.4 Hz, 1H), 8.27 (dd, J = 8.0, 1.6 Hz, 2H), 8.02 (s, 1H), 7.55-7.24 (m, 1H), 7.20 (dd, J = 8.0, 4.6 Hz, 1H), 4.28 (s, 2H), 1.81 (m, J = 16.6, 8.2 Hz, 1H), 1.65-1.48 (m, 2H), 1.28 (s, 3H), 0.94 (dd, J = 14.3, 6.6 Hz, 6H).

### Example 47: Synthesis of (S)-1-{4-(1H-pyrrolo[2,3-b]pyridin-3-yl)-2-(trifluoromethyl)phenoxy}-2,4-dimethylpenta n-2-amine (Compound 47)

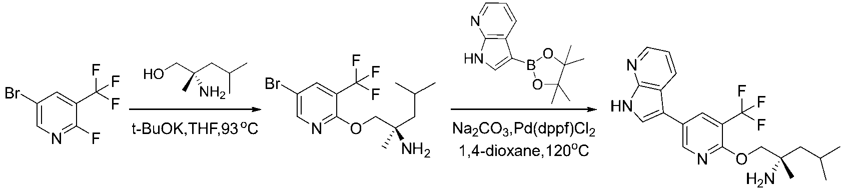

### Step 1: Synthesis of (S)-1-[4-bromo-2-(trifluoromethyl)phenoxy]-2,4-dimethylpentan-2-amine

A compound (S)-2-amino-2,4-dimethylpentan-1-ol (200 mg, 1.52 mmol, 0.95 eq) was added to tetrahydrofuran (5.0 mL), followed by the addition of potassium tert-butoxide (450 mg, 4.0 mmol, 2.5 eq) and 5-bromo-2-fluorobenzotrifluoride (390 mg, 1.60 mmol, 1.0 eq). The mixture was reacted at room temperature for 2 hours. After the reaction was completed, the mixture was filtered, and the filtrate was concentrated and purified by normal-phase column chromatography (petroleum ether:ethyl acetate = 3:1) to obtain (S)-1-[4-bromo-2-(trifluoromethyl)phenoxy]-2,4-dimethylpentan-2-amine as a colorless oily product (240 mg, yield 44.50%). LCMS(TOF MS ES+)m/z [M+H]+: 354/356. 1H NMR (400 MHz, CDCl3) δ 7.67 (d, J = 2.5 Hz, 1H), 7.57 (dd, J = 8.8, 2.5 Hz, 1H), 6.84 (d, J = 8.8 Hz, 1H), 3.82 - 3.68 (m, 2H), 1.85-1.68 (m, J = 6.4 Hz, 1H), 1.47 (dd, J = 5.7, 4.0 Hz, 2H), 1.22 (s, 3H), 0.96 (dd, J = 9.9, 6.7 Hz, 6H).

### Step 2: Synthesis of (S)-1-{4-(1H-pyrrolo[2,3-b]pyridin-3-yl)-2-(trifluoromethyl)phenoxy}-2,4-dimethylpenta n-2-amine

(S)-1-[4-bromo-2-(trifluoromethyl)phenoxy]-2,4-dimethylpentan-2-amine (92 mg, 0.26 mmol, 1.0 eq), 7-azaindole-3-boronic acid pinacol ester (63 mg, 0.26 mmol, 1.0 eq), sodium carbonate (110 mg, 1.04 mmol, 4.0 eq), and [1,1'-bis(diphenylphosphino)ferrocene]palladium dichloride (19 mg, 0.026 mmol, 0.10 eq) were added to 1,4-dioxane (3.0 mL). The mixture was reacted at 120°C for 8 hours under a nitrogen atmosphere. After the reaction was completed, the mixture was filtered, and the filtrate was purified by reverse-phase chromatography on a C18 column (sodium bicarbonate system) to obtain (S)-1-{4-(1H-pyrrolo[2,3-b]pyridin-3-yl)-2-(trifluoromethyl)phenoxy}-2,4-dimethylpentan-2 -amine as a brown solid (3.1 mg, yield 3.05%). LCMS(TOF MS ES+)m/z [M+H]+: 392.2213. 1H NMR (400 MHz, MeOD) δ 8.30-8.25(m, 2H), 7.93-7.85 (m, 2H), 7.68 (s, 1H), 7.28 (d, J = 8.5 Hz, 1H), 7.24 (dd, J = 7.8, 5.0 Hz, 1H), 4.01-3.90 (m, 2H), 1.90 - 1.80 (m, 1H), 1.58 (m, J = 14.2, 5.5 Hz, 2H), 1.29 (s, 3H), 1.02 (t, J = 7.0 Hz, 6H).

The compounds in Table 1 were obtained following the methods described in Examples 1 and 2.

**Table 1: Structures and characterization of other compounds**

| Exampl e | Compoun d number | Chemical structural formula | LC-MS(ESI)m/z [M+H]⁺ | Exampl e | Compound number | Chemical structural formula | LC-MS( ESI)m/z[ M+H]⁺ |
|---|---|---|---|---|---|---|---|
| 3 | 3 | 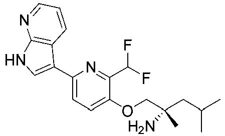 | 375.2; ¹H NMR (400 MHz, MeOD) δ 8.95 (m, J = 8.0, 1.7 Hz, 1H), 8.55 (s, 1H), 8.29 (d, J = 4.7 Hz, 1H), 8.06 (d, J = 1.7 Hz, 1H), 7.99 (d, J = 8.8 Hz, 1H), 7.69 (d, J = 8.9 Hz, 1H), 7.34 - 7.03 (m, 2H), 4.29 - 4.23 (m, 1H), 4.21 - 4.14 (m, 1H), 1.95 - 1.83 (m, 2H), 1.77 - 1.67 (m, 1H), 1.53 (d, J = 1.7 Hz, 3H), 1.08 (m, J = 15.5, 6.5, 1.8 Hz, 6H). | 23 | 23 | 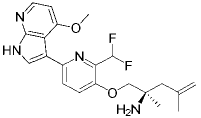 | 403.2 |
| 4 | 4 | 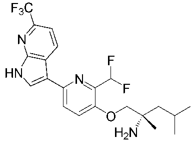 | 443.2 | 24 | 24 | 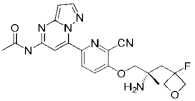 | 439.2 |
| 5 | 5 | 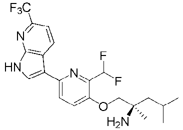 | 443.2 | 25 | 25 | 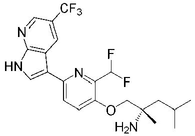 | 443.2 |
| 6 | 6 | 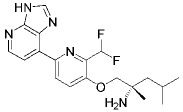 | 376.2 | 27 | 27 | 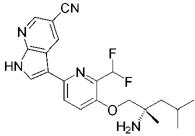 | 400.2 |
| 7 | 7 | 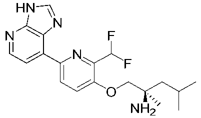 | 376.2 | 29 | 29 | 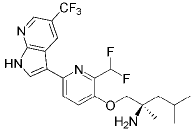 | 443.2 |
| 8 | 8 | 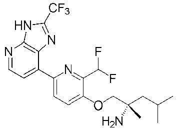 | 444.2 | 30 | 30 | 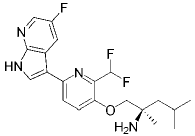 | 393.2 |
| 9 | 9 | 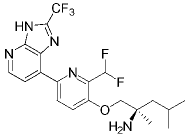 | 444.2 | 31 | 31 | 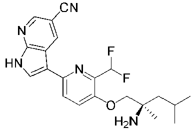 | 400.2 |
| 10 | 10 | 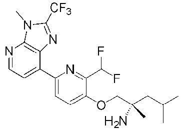 | 458.2 | 32 | 32 | 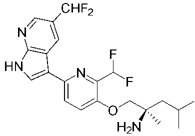 | 425.2 |
| 11 | 11 | 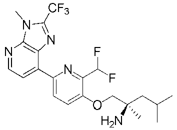 | 458.2 | 33 | 33 | 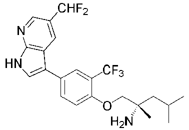 | 442.2 |
| 12 | 12 | 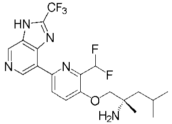 | 444.2 | 34 | 34 | 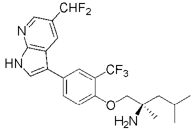 | 442.2 |
| 13 | 13 | 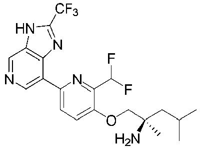 | 444.2 | 35 | 35 | 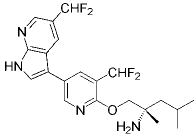 | 425.2 |
| 14 | 14 | 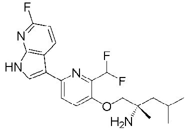 | 393.2 | 36 | 36 | 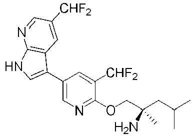 | 425.2 |
| 15 | 15 | 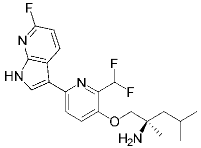 | 393.2 | 37 | 37 | 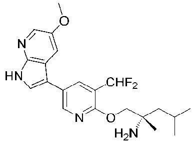 | 405.2 |
| 18 | 18 | 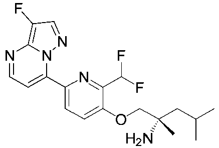 | 394.2 | 38 | 38 | 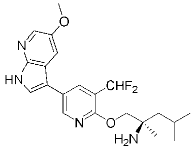 | 405.2 |
| 19 | 19 | 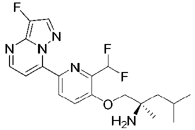 | 394.2 | 39 | 39 | 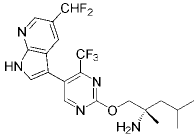 | 444.2 |
| 20 | 20 | 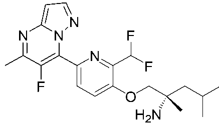 | 408.2 | 40 | 40 | 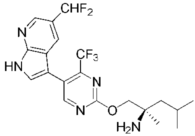 | 444.2 |
| 21 | 21 | 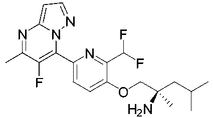 | 408.2 | 41 | 41 | 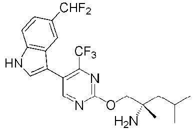 | 443.2 |
| 22 | 22 | 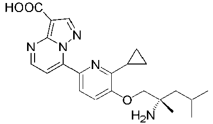 | 410.2 | 42 | 42 | 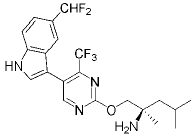 | 443.2 |

### Experimental Example 1: Test on the binding ability of the compound of the present invention to AAK1 protein

Experimental objective: To detect the binding ability of compounds to AAK1 protein by means of a CETSA experiment.

Background principle: The CETSA experiment is a molecular detection means for measuring the affinity between drugs and target proteins. The principle is that when a drug binds to a target protein, it makes the structure of the target protein more stable. When a candidate drug is co-incubated with a target protein, if the candidate drug is an inhibitor of AAK1, it can bind to AAK1. During the heating treatment of the sample, the AAK1 protein will be more stable, and thus the AAK1 protein can be more easily detected by Western blot experiment. On the contrary, the stability of the AAK1 protein after heating will be poorer, and the amount of the detected protein will be lower. In this way, the binding ability of the drug to the target protein can be evaluated, which is used for the screening of AAK1 protein inhibitors.

### Specific experimental procedure:

Expression of AAK1 (30-330) protein: The AAK1 sequence (residues 30-330) was cloned into the pET24N vector with an N-terminal (His)6x tag and a tobacco etch virus (TEV) protease cleavage site. The constructed vector was transformed into *Escherichia coli* BL21(DE3). When the bacteria were cultured with shaking to reach an OD value of 0.6, 1 mM IPTG was added. Then, the bacteria were cultured at 12°C for 16 h. The bacterial cells were collected and subjected to ultrasonic disruption. The supernatant was obtained by centrifugation, and the protein was purified using Ni-NTA. After dialysis, the concentration and purity of the protein were determined.

CETSA sample preparation: Samples were incubated with candidate drugs, control drugs, and control reagents separately for 30 minutes. Each group of samples was heated at approximately 10 pre-set temperature points. After being returned to room temperature, the samples were centrifuged at 20000 g, and the supernatants were collected. Protein samples were denatured by heating at 100°C for 10 minutes with a sample buffer. After the samples were returned to room temperature, Western blot detection was performed on them; the protein loading amount was controlled at 20 µg. After the melting temperature was determined, the compound was set with a concentration gradient, generally 9 points, and the samples were incubated. The same operations were followed, and Western blot detection was conducted. Protein electrophoresis: The voltage for the stacking gel was set at 60 V, and that for the separating gel was set at 120 V. After electrophoresis, electrotransfer was initiated. Electrotransfer conditions were set at 250 mA for 2 h. Blocking with 5% BSA was performed for 1 h. A specific primary antibody was added, and incubation was carried out overnight at 4°C on a shaker. Washing with TBST was conducted 4 times, with 2.5 minutes each time. A secondary antibody was incubated on a shaker at room temperature for 1 h. Washing with TBST was conducted 4 times, with 2.5 minutes each time. ECL was used for development to detect the expression levels of AAK1 protein in different groups and at each temperature point. Western blot bands were processed and converted using Image J and GraphPad software to calculate EC50.

EC50 (concentration for 50% of maximal effect) refers to the drug concentration that can produce an effective response in 50% of individuals. The AAK1 inhibitor BMS-986176/LX-9211 from Bristol-Myers Squibb Company was used as a positive reference compound, and its preparation method was referenced from Example 123 in the patent document CN106458994A.

The structure of BMS-986176/LX-9211 is as follows:

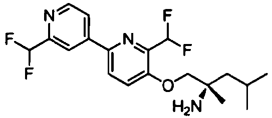

The test results are shown in Table 2 below. The EC50 values of each compound are classified as follows:
"+" indicates an EC50 value > 1 µM;
"++" indicates 100 nM < an EC50 value < 1 µM;
"+++" indicates 10 nM < an EC50 value < 100 nM;
"++++" indicates an EC50 value < 10 nM.

**Table 2: EC50 results**

| Compound number | EC50 | Compound number | EC50 | Compound number | EC50 | Compound number | EC50 |
|---|---|---|---|---|---|---|---|
| 1 | +++ | 12 | +++ | 23 | ++ | 34 | +++ |
| 2 | +++ | 13 | +++ | 24 | ++ | 35 | +++ |
| 3 | +++ | 14 | +++ | 25 | +++ | 36 | ++ |
| 4 | +++ | 15 | +++ | 26 | +++ | 37 | +++ |
| 5 | +++ | 16 | +++ | 27 | +++ | 38 | +++ |
| 6 | +++ | 17 | +++ | 28 | +++ | 39 | +++ |
| 7 | ++ | 18 | +++ | 29 | ++ | 40 | +++ |
| 8 | +++ | 19 | +++ | 30 | +++ | 41 | ++ |
| 9 | +++ | 20 | +++ | 31 | +++ | 42 | +++ |
| 10 | +++ | 21 | ++ | 32 | +++ | 43 | ++++ |
| 11 | +++ | 22 | ++ | 33 | +++ | 44 | ++++ |
| BMS-986176 | ++ | | | | | 45 | ++++ |

Experimental results indicate that the compounds of the present invention exhibit strong binding ability to the functional domain of the AAK1 protein kinase, with in vitro binding capacity comparable to or stronger than that of the positive control compound BMS-986176. This suggests that the compounds of the present invention have the potential to act as AAK1 protein inhibitors.

### Experimental Example 2: Determination of the effect of the compound of the present invention on AP2M1 phosphorylation in cells

Experimental objective: The objective of this test example is to determine the effect of compounds on AP2M1 phosphorylation in cells.

Background principle: Adaptor-associated kinase 1 (AAK1), also known as AP2-associated kinase 1, is a 104 kDa serine/threonine kinase. AAK1 interacts with the adaptor protein 2 (AP2) complex and phosphorylates the threonine residue in the micro-subunit of AP2 (AP2M1), thereby increasing its binding affinity for certain tyrosine-based or dileucine-based sorting signals of some membrane receptors. The inhibitory effect of compounds on AAK1 was evaluated by detecting the phosphorylation level of AP2M1 in cells, so as to screen candidate compounds.

### Specific experimental procedure:

293T cells were seeded in T25 flasks, so that the confluency would reach 60%-80% the next day. On the second day, each T25 flask was co-transfected with 16 µg of plasmids (1 µg of AAK1/HA/pIRES and 15 µg of Flag/AP2MI/pcDNA) using a Lip2000 transfection reagent. On the third day, the cells were digested and resuspended, centrifuged at 1000 rpm for 5 minutes, and then seeded into 24-well plates at 450 µl per well (2 × 10⁵ cells/well). Gradient dilutions of the compounds to be tested were prepared, 50 µl was added to each well, mixed thoroughly, and incubated at 37°C for 3 h. DMSO was set as a control, and cells without plasmid transfection were set as a blank control. After incubation, the cells were collected and washed twice with PBS. The cells were lysed on ice for 30 minutes using a cell lysis buffer containing the protease inhibitor PMSF. The concentration of protein samples was determined using a BCA kit. Protein electrophoresis: The voltage for the stacking gel was set at 60 V, and that for the separating gel was set at 120 V. After electrophoresis, electrotransfer was initiated. Electrotransfer conditions were set at 250 mA for 2 h. Blocking with 5% BSA was performed for 1 h. A specific primary antibody, anti-p-AP2M1, was added, and incubation was carried out overnight at 4°C on a shaker. Washing with TBST was conducted 4 times, with 2.5 minutes each time. A secondary antibody was incubated on a shaker at room temperature for 1 h. Washing with TBST was conducted 4 times, with 2.5 minutes each time. ECL was used for development to detect the expression level of p-AP2M1. Western blot bands were processed and converted using Image J and GraphPad software to calculate IC50.

IC50 (half maximal inhibitory concentration) refers to the half-inhibitory concentration of the measured antagonist. It can indicate the concentration of a certain drug or substance (inhibitor) that achieves half of the inhibition effect on certain biological processes (or certain substances involved in these processes, such as enzymes, cellular receptors, or microorganisms). Compound BMS-986176, as described above, was used as a positive reference compound.

The test results are shown in Table 3 below. The IC50 values of each compound are classified as follows:
"+" indicates an IC50 value > 1 µM;
"++" indicates 100 nM < an IC50 value < 1 µM;
"+++" indicates 10 nM < an IC50 value < 100 nM;
"++++" indicates an IC50 value < 10 nM.

The test results are shown in Table 3 below:

**Table 3: IC50 experimental data**

| Compound number | IC50 | Compound number | IC50 | Compound number | IC50 | Compound number | IC50 |
|---|---|---|---|---|---|---|---|
| 1 | +++ | 12 | +++ | 23 | +++ | 34 | +++ |
| 2 | +++ | 13 | +++ | 24 | +++ | 35 | ++ |
| 3 | +++ | 14 | +++ | 25 | +++ | 36 | +++ |
| 4 | +++ | 15 | +++ | 26 | +++ | 37 | +++ |
| 5 | +++ | 16 | +++ | 27 | ++ | 38 | +++ |
| 6 | +++ | 17 | ++ | 28 | +++ | 39 | +++ |
| 7 | +++ | 18 | +++ | 29 | +++ | 40 | +++ |
| 8 | +++ | 19 | +++ | 30 | +++ | 41 | ++ |
| 9 | +++ | 20 | +++ | 31 | +++ | 42 | +++ |
| 10 | +++ | 21 | +++ | 32 | ++ | 43 | ++++ |
| 11 | +++ | 22 | +++ | 33 | +++ | 44 | ++++ |
| BMS-986176 | ++ | | | | | 45 | ++++ |

The results show that the compounds of the present invention can inhibit the phosphorylation level of AP2M1 in cells, with IC50 values reaching the nanomolar (nM) level, which are comparable to or stronger than that of the positive drug. This strong inhibitory effect has significant therapeutic implications for the treatment of disorders or diseases related to AAK1 inhibition.

### Experimental Example 3: Test on the effect of the compound of the present invention on AAK1 kinase function

Experimental objective: The objective of this test example is to test the effect of compounds on AAK1 kinase function.

Background principle: AAK1 phosphorylates the threonine residue in the micro-subunit of AP2 (AP2M1). In this process, ATP provides phosphate groups to generate ADP. Therefore, the activity of AAK1 kinase can be evaluated by detecting the amount of ADP generated.

### Specific experimental procedure:

Expression of AAK1 (30-330) protein: The AAK1 sequence (residues 30-330) was cloned into the pET24N vector with an N-terminal (His)6x tag and a tobacco etch virus (TEV) protease cleavage site. The constructed vector was transformed into *Escherichia coli* BL21(DE3). When the bacteria were cultured with shaking to reach an OD value of 0.6, 1 mM IPTG was added. Then, the bacteria were cultured at 12°C for 16 h. The bacterial cells were collected and subjected to ultrasonic disruption. The supernatant was obtained by centrifugation, and the protein was purified using Ni-NTA. After dialysis, the concentration and purity of the protein were determined.

AAK1 kinase assay: The purified AAK1 protein was added to the buffer to be tested (20 mM Tris, pH 7.80, 10 mM MgCl₂, 1 mM DTT, 0.01% Tween 20), followed by the addition of the substrate (Aha-KEEQSQITSQVTGQIGWR-NH2 and ATP) and the compound to be tested. Final concentrations of reagents in the reaction system: 3.5 nM for AAK1, 1.5 µM for Aha-KEEQSQITSQVTGQIGWR-NH2, and 22 µM for ATP. The reaction mixture was incubated in a metal bath at 25°C for 3 h. The reaction was terminated by adding 1% SDS, and the amount of ADP generated was detected by HPLC. IC50 values were calculated using GraphPad software. Compounds were screened by comparison with the positive drug BMS-986176.

The test results are shown in Table 4 below:

**Table 4: IC50 experimental data**

| Compound number | IC50 (nM) | Compound number | IC50 (nM) | Compound number | IC50 (nM) |
|---|---|---|---|---|---|
| 1 | 32.1 | 16 | 87.2 | 31 | 102.1 |
| 2 | 28.3 | 17 | 56.9 | 32 | 67.8 |
| 3 | 21.2 | 18 | 42.5 | 33 | 69.5 |
| 4 | 45.9 | 19 | 87.3 | 34 | 89.2 |
| 5 | 86.1 | 20 | 101.6 | 35 | 113.4 |
| 6 | 75.2 | 21 | 190.2 | 36 | 147.6 |
| 7 | 100.5 | 22 | 170.3 | 37 | 123.7 |
| 8 | 129.3 | 23 | 99.6 | 38 | 160.2 |
| 9 | 99.2 | 24 | 107.4 | 39 | 101.3 |
| 10 | 152.1 | 25 | 118.5 | 40 | 99.5 |
| 11 | 123.4 | 26 | 86.3 | 41 | 96.7 |
| 12 | 99.2 | 27 | 57.8 | 42 | 78.4 |
| 13 | 167.8 | 28 | 65.3 | 43 | 12.1 |
| 14 | 102.3 | 29 | 72.1 | 44 | 8.2 |
| 15 | 111.2 | 30 | 88.9 | 45 | 6.5 |
| BMS-986176 | 260.3 | | | | |

The results indicate that the compounds of the present invention exhibit potent inhibitory effects on the function of AAK1 protein kinase, with all IC50 values reaching the nanomolar (nM) level, which are comparable to or lower than that of the positive drug. This strong inhibitory effect has significant therapeutic implications for the treatment of disorders or diseases related to AAK1 inhibition.

### Experimental Example 4: In vitro pharmacokinetic test on the compound of the present invention in SD rats

Experimental objective: The objective of this experimental example is to test the pharmacokinetic profile of compounds in SD rats.

Background principle: Non-clinical pharmacokinetic studies reveal the dynamic change rules of drugs in the body, obtain basic pharmacokinetic parameters of drugs, and elucidate the processes and characteristics of drug absorption, distribution, metabolism, and excretion through in vivo and in vitro studies in animals as well as in vitro studies in humans. Non-clinical pharmacokinetic studies play an important role in the evaluation process of new drug research and development. In pharmacodynamic and toxicological evaluations, the concentration data of drugs or active metabolites and their related pharmacokinetic parameters are the foundation for generating, determining, or elucidating the magnitude of efficacy or toxicity, and can provide a basis for the effects (efficacy or toxicity) of drugs on target organs.

### Specific experimental procedure:

Preparation of compounds to be tested: Vehicle: 40% PEG300, 15% Tween 80, 10% ethanol, 35% water, prepared on the day of administration;
Experimental animals: Male SD rats, SPF grade, 18 animals;
Experimental design:

**Table 5: Experimental design**

| **Group** | **Test compound** | **Number of animals Male** | **Dose** | **Concentration** | **Route of administration** | **Sample collected** |
|---|---|---|---|---|---|---|
| | | | **(mg/kg)** | **(mg/mL)** | | |
| 1 | Compound 26 | 3 | 2 | 0.4 | IV, single dose | Plasma |
| 2 | | 3 | 1.5 | 0.15 | PO*, single dose | Plasma |
| 3 | | 3 | 3 | 0.3 | PO*, single dose | Plasma |
| 4 | | 3 | 6 | 0.6 | PO*, single dose | Plasma |
| 5 | BMS-986176 | 3 | 2 | 0.4 | IV, single dose | Plasma |
| 6 | | 3 | 3 | 0.3 | PO*, single dose | Plasma |

| | | | | | | |
|---|---|---|---|---|---|---|
| Note: * The animals were fasted overnight (10-14 hours) before administration and were fed 4 hours after administration. | | | | | | |

Route of administration: The animals were weighed before administration, and the dose was calculated based on their body weight. Administration was performed via intravenous injection and oral gavage.

Blood collection time points: After administration, for IV group: 0.083 h, 0.25 h, 0.5 h, 1 h, 2 h, 4 h, 8 h, and 24 h; for PO group: 0.25 h, 0.5 h, 1 h, 2 h, 4 h, 6 h, 8 h, and 24 h. Blood was collected via the jugular vein, with approximately 0.2 mL collected for each sample. The blood samples were anticoagulated with K2-EDTA and placed on ice after collection.

Plasma sample processing: After collection, the blood samples were placed on ice and centrifuged within 1 hour to separate plasma (centrifugation conditions: 6800g, 6 minutes, 2-8°C). The plasma samples were stored in a -80°C refrigerator before analysis.

Result analysis: Pharmacokinetic parameters were calculated using Phoenix WinNonlin 7.0 based on the plasma concentration data at different time points. Parameters including AUC0-t, AUC0-∞, MRT0-∞, Cmax, Tmax, and T1/2 were provided, along with their mean values and standard deviations.

Experimental results are shown in Table 6 below:

**Table 6: Pharmacokinetic data of Compound 26**

| **Compound** | | **Compound 26** | **BMS-986176** |
|---|---|---|---|
| Dose level | mg/kg | 3.00 | 3.00 |
| T_{1/2} | h | 6.98 | 6.32 |
| Tₘₐₓ | h | 3.10 | 3.33 |
| Cₘₐₓ | ng/mL | 325.23 | 85.60 |
| AUC₍₀₋ₜ₎ | h*ng/mL | 4012.50 | 1069.29 |
| AUC_{(0-∞)} | h*ng/mL | 4126.32 | 1159.48 |
| MRT₍₀₋ₜ₎ | h | 8.09 | 7.52 |
| MRT_{(0-∞)} | h | 10.93 | 9.50 |

Experimental conclusion: The pharmacokinetic data of Compound 26 in SD rats show that, compared with the positive control, the half-life and time to peak (Tmax) of Compound 26 in SD rats are comparable, but the peak concentration (Cmax) and in vivo exposure are approximately four times those of the positive control. In general, the in vivo pharmacokinetic data of Compound 26 in rats are significantly superior to those of BMS-986176.

### Experimental Example 5: Efficacy test on the compound of the present invention in diabetic peripheral neuropathic pain (DPNP) in SD rats

Experimental objective: This experiment aims to investigate the analgesic effect of test compounds on streptozotocin (STZ)-induced peripheral neuropathic pain (DPNP) in diabetic rat models and the brain-blood distribution of the compounds associated with the efficacy, via single oral gavage (ig.) administration.

Experimental methods: After the adaptation period, the rats were tested for baseline plantar pain threshold using the Von Frey (Up-Down method) before modeling. Subsequently, except for the blank control group, all other rats were fasted for 16 hours with free access to water and then intraperitoneally injected with streptozotocin for modeling (65 mg/kg; 2 mL/kg). One week after modeling, blood was collected from the tail tip to detect blood glucose levels after a 4-hour fast. Rats with blood glucose levels higher than 16.7 mmol/L were considered diabetic.

After successful modeling, the baseline plantar pain threshold of the rats was tested using the Von Frey (UP-Down method) as the baseline pain threshold before administration, and the rats were evenly grouped according to their pain thresholds. The groups included a blank control group, a model group, a positive control group (1 mg/kg), a Compound 26 (0.5 mg/kg) group, and a Compound 26 (1 mg/kg) group. The next day, the rats were administered the vehicle or corresponding drugs. At 2 h, 3 h, 4 h, 5 h, and 6 h after administration, the pain threshold of the plantar region on the affected side of rats was determined using Von Frey filaments to calculate the pain threshold improvement rate of the test compounds against neuropathic pain in diabetic rats, and to observe the time-effect relationship. After the experiment, rats from groups G3-G5 (numbers 1-3 per group) were sacrificed, and plasma and brain tissue samples were collected for testing.

The experimental results are shown in Tables 7, 8, 9, and FIG. 1 of the specification. Before modeling, the baseline pain threshold of rats in all groups was 15 g. After modeling, except for the blank control group, the pain threshold of rats in the other groups decreased to approximately 7.0 g, showing a statistically significant difference compared with the blank control group (p < 0.01).

**Table 7: Effect of single oral gavage (ig.) of Compound 26 on the pain threshold of peripheral neuropathic pain in diabetic rats (x±s, n=5)**

| Group | MWT (g) | | | | | | |
|---|---|---|---|---|---|---|---|
| | Baseline | After modeling | 2h | 3 h | 4 h | 5 h | 6h |
| Control | 15.00±0. 00 | 15.00±0.0 0 | 15.00±0.00 | 15.00±0.0 0 | 15.00 ±0.00 | 15.00 ±0.00 | 15.00 ±0.00 |
| Model | 15.00±0. 00 | 6.89±2.18 ## | 3.68±0.90# # | 4.34±0.00 ## | 4.03± 0.69# # | 4.03± 0.69# # | 3.66± 0.94# # |
| BMS-9861 76 1mg/kg | 15.00±0. 00 | 7.00±1.83 | 10.38±2.59 ** | 7.51±4.29 | 5.06± 2.76 | 4.82± 1.07 | 4.63± 0.64 |
| Compound 26 (0.5 mg/kg) | 15.00±0. 00 | 6.97±2.16 | 7.50±2.37* | 9.63±3.40 * | 6.23± 2.10 | 5.61± 3.00 | 4.82± 1.07 |
| Compound 26 (1 mg/kg) | 15.00±0. 00 | 7.28±2.09 | 12.72±3.12 ** | 10.56±4.1 9* | 9.96± 4.93 | 5.10± 1.55 | 4.05± 0.66 |

| | | | | | | | |
|---|---|---|---|---|---|---|---|
| *## p<0.01* vs Control* *p<*0.05,*** p<*0.01 vs Model | | | | | | | |

**Table 8: Effect of single oral gavage (ig.) of Compound 26 on the pain threshold improvement rate of peripheral neuropathic pain in diabetic rats (x±s, n=5)**

| Group | Pain threshold improvement rate | | | | |
|---|---|---|---|---|---|
| | 2h | 3 h | 4h | 5 h | 6h |
| BMS-986176 (1 mg/kg) | 64.51 | 42.24 | 20.35 | 16.32 | 20.85 |
| Compound 26 (0.5 mg/kg) | 50.87 | 54.93 | 35.29 | 28.18 | 24.02 |
| Compound 26 (1 mg/kg) | 71.05 | 58.89 | 59.53 | 20.88 | 9.51 |

**Table 9: Blood-brain distribution of compounds in DPNP model rats 6 h after intragastric administration**

| Compound | Dose (mg/kg) | Plasma concentration (ng/mL) | Brain tissue concentration (ng/mL) | Brain tissue concentration* (ng/g) | Brain/plasma ratio |
|---|---|---|---|---|---|
| BMS-986176 | 1 | 34.38 | 26.94 | 296.36 | 8.62 |
| Compound 26 | 0.5 | 38.45 | 62.53 | 687.83 | 17.89 |
| Compound 26 | 1 | 75.26 | 120.14 | 1321.57 | 17.56 |

Experimental conclusion: In this experiment on the analgesic effect of the test compounds on peripheral neuropathic pain in diabetic rats, (1) after 2 h of administering the test compounds BMS-986176 (1 mg/kg), Compound 26 (0.5 mg/kg), and Compound 26 (1 mg/kg) separately, the pain threshold of the model animals is significantly increased, and the duration of analgesia can last until 4 h after administration; (2) both the analgesic effect and the duration of action of Compound 26 were superior to those of the positive control BMS-986176; and (3) compared with the positive control BMS-986176, the compound has better brain permeability.

## Claims

1. A compound of formula (I), a stereoisomer thereof, or a pharmaceutically acceptable salt thereof: 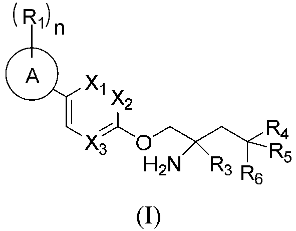 wherein
X₁, X₂, and X₃ are each independently selected from N or CR₂;
a ring A is selected from: 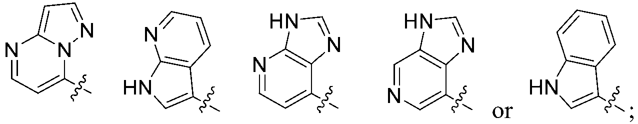
R₁, R₂, and R₃ are each independently selected from hydrogen, amino, -CO₂H, halogen, fluoromethyl, difluoromethyl, trifluoromethyl, cyano, formamido, C₁₋₆ alkyl, deuterated C₁₋₆ alkyl, halogenated C₁₋₆ alkyl, C₁₋₆ alkoxy, deuterated C₁₋₆ alkoxy, halogenated C₁₋₆ alkoxy, hydroxy C₁₋₆ alkyl, C₃₋₆ cycloalkyl, or 4-6 membered heterocycloalkyl containing 1-3 heteroatoms selected from N, S, or O;
R₄, R₅, and R₆ are each independently selected from hydrogen, deuterium, halogen, amino, cyano, hydroxy, alkenyl, fluoromethyl, difluoromethyl, trifluoromethyl, C₁₋₆ alkyl, halogenated C₁₋₆ alkyl, C₁₋₆ alkoxy, halogenated C₁₋₆ alkoxy, deuterated C₁₋₆ alkyl, deuterated C₁₋₆ alkoxy, C₃₋₆ cycloalkyl, hydroxy C₁₋₆ alkyl, or 4-6 membered heterocycloalkyl containing 1-3 heteroatoms selected from N, S, or O;
or R₄ and R₅, together with carbon atoms to which they are each attached, form C₃₋₆ cycloalkyl, 4-6 membered heterocycloalkyl containing 1-3 heteroatoms selected from N, S, or O, or a double bond; and
n is selected from 0, 1, 2, 3, or 4, and when the n is greater than 1, R₁ is the same or different.

2. The compound, the stereoisomer thereof, or the pharmaceutically acceptable salt thereof according to claim 1, wherein R₁ is selected from hydrogen, fluorine, chlorine, -CO₂H, cyano, methoxy, methyl, difluoromethyl, trifluoromethyl, or formamido.

3. The compound, the stereoisomer thereof, or the pharmaceutically acceptable salt thereof according to claim 1, wherein R₂ is selected from hydrogen, methyl, methoxy, cyano, difluoromethyl, trifluoromethyl, or cyclopropyl.

4. The compound, the stereoisomer thereof, or the pharmaceutically acceptable salt thereof according to claim 1, wherein R₃ is methyl.

5. The compound, the stereoisomer thereof, or the pharmaceutically acceptable salt thereof according to claim 1, wherein R₄, R₅, and R₆ are each independently selected from hydrogen, methyl, halogen, alkenyl, or 4-6 membered heterocycloalkyl containing 1-3 heteroatoms selected from N, S, or O.

6. The compound, the stereoisomer thereof, or the pharmaceutically acceptable salt thereof according to claim 1, wherein R₄ and R₅, together with the carbon atoms to which they are each attached, form 4-6 membered heterocycloalkyl containing 1-3 heteroatoms selected from N, S, or O, or a double bond.

7. The compound, the stereoisomer thereof, or the pharmaceutically acceptable salt thereof according to any one of claims 4-6, wherein 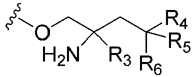 is selected from the following groups:

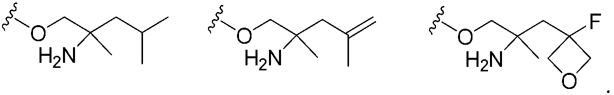

8. A compound of formula (I-1), a stereoisomer thereof, or a pharmaceutically acceptable salt thereof: 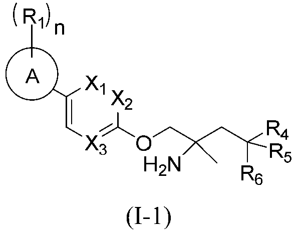 wherein R₁, a ring A, n, R₄, R₅, R₆, X₁, X₂, and X₃ are as defined in claim 1.

9. A compound of formula (I-2), a stereoisomer thereof, or a pharmaceutically acceptable salt thereof: 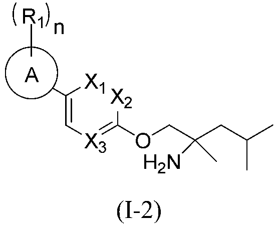 wherein X₁, X₂, X₃, R₁, a ring A, and n are as defined in claim 1.

10. A compound of formula (I-3), (I-4), (I-5), (I-6), (I-7), or (I-8), a stereoisomer thereof, or a pharmaceutically acceptable salt thereof: 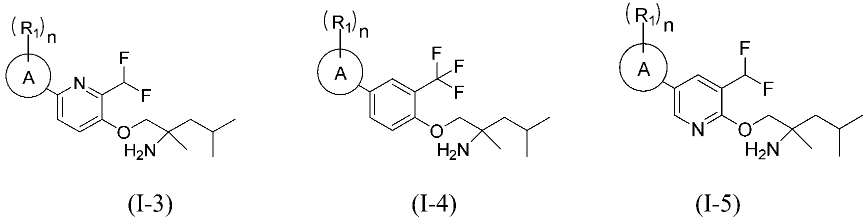 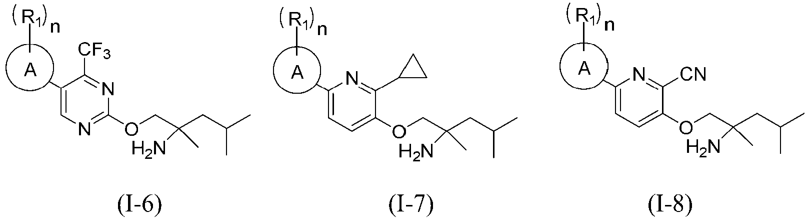 wherein R₁, a ring A, and n are as defined in claim 1.

11. The compound, the stereoisomer thereof, or the pharmaceutically acceptable salt thereof according to claim 1, wherein the compound is selected from the following structures:

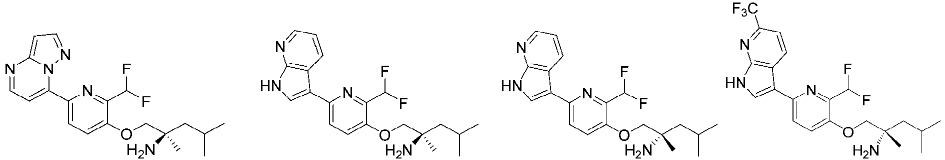

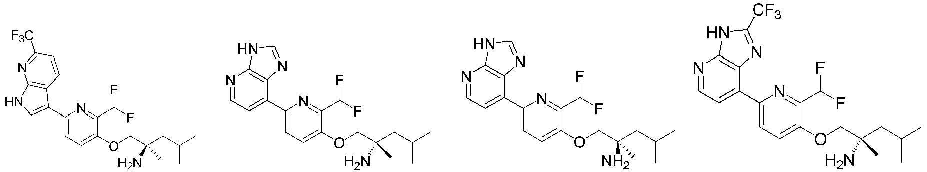

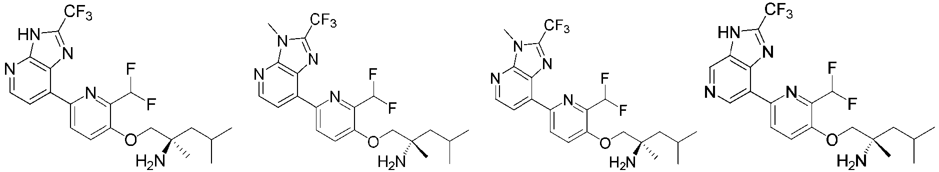

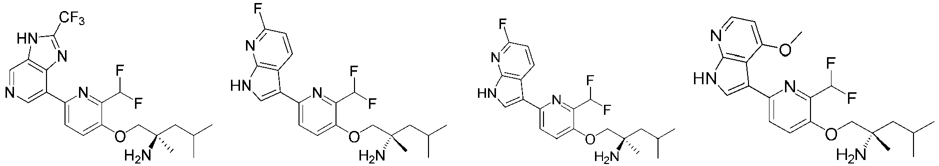

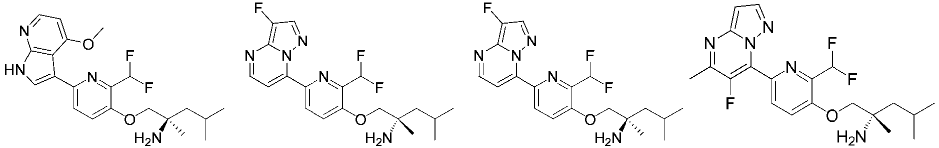

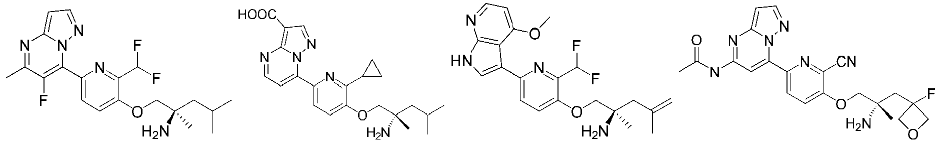

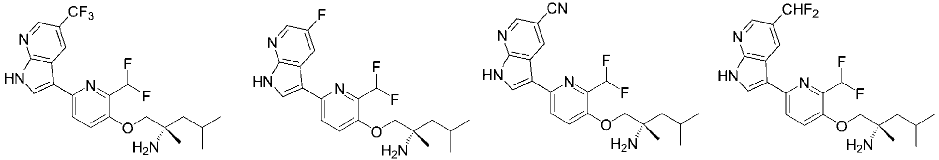

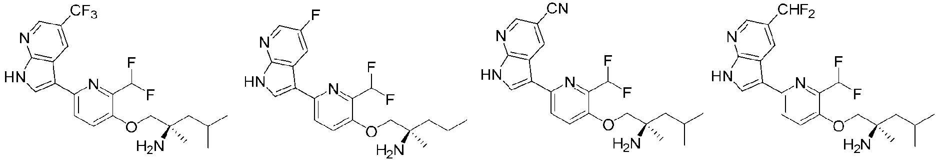

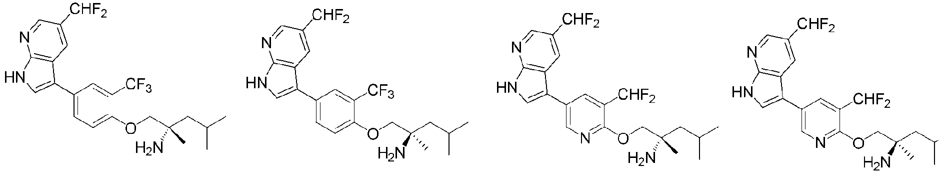

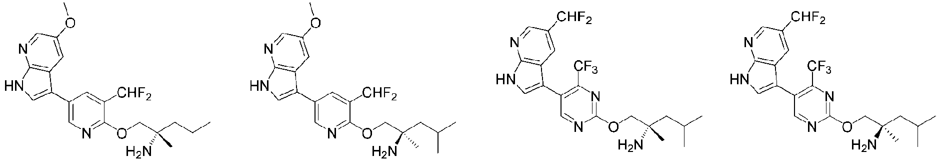

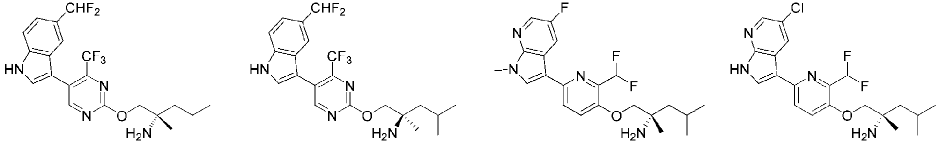

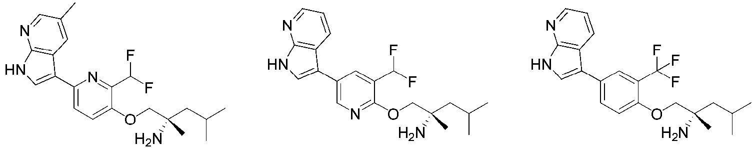

12. A pharmaceutical composition, comprising the compound, the stereoisomer thereof, or the pharmaceutically acceptable salt thereof according to any one of claims 1-11, and a pharmaceutically acceptable carrier.

13. Use of the compound, the stereoisomer thereof, or the pharmaceutically acceptable salt thereof according to any one of claims 1-11, or the pharmaceutical composition according to claim 12 in preparation of a medicament for treating a disease or a disorder mediated by activity of adaptor associated kinase 1, and
the disease or the disorder is preferably selected from Alzheimer's disease, bipolar disorder, Parkinson's disease, schizophrenia, diabetic peripheral neuropathic pain, or postherpetic neuralgia.
